(19) Europäisches Patentamt
European Patent Office
Office européen des brevets

(11) **EP 2 714 128 B1**

(12) **EUROPÄISCHE PATENTSCHRIFT**

(45) Veröffentlichungstag und Bekanntmachung des Hinweises auf die Patenterteilung:
**17.08.2016 Patentblatt 2016/33**

(21) Anmeldenummer: **12726739.1**

(22) Anmeldetag: **22.05.2012**

(51) Int Cl.:
***A61M 1/16*** *(2006.01)*

(86) Internationale Anmeldenummer:
**PCT/EP2012/002167**

(87) Internationale Veröffentlichungsnummer:
**WO 2012/159734 (29.11.2012 Gazette 2012/48)**

(54) **VORRICHTUNG UND VERFAHREN ZUR ERKENNUNG EINES BETRIEBSZUSTANDES EINER EXTRAKORPORALEN BLUTBEHANDLUNG**

DEVICE AND METHOD FOR RECOGNIZING AN OPERATING STATE OF AN EXTRA-CORPOREAL BLOOD TREATMENT

DISPOSITIF ET PROCÉDÉ POUR LA DÉTECTION D'UN ÉTAT DE FONCTIONNEMENT D'UN TRAITEMENT DE SANG EXTRACORPOREL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **23.05.2011 DE 102011102962**
**23.05.2011 US 201161489037 P**

(43) Veröffentlichungstag der Anmeldung:
**09.04.2014 Patentblatt 2014/15**

(73) Patentinhaber: **Fresenius Medical Care Deutschland GmbH**
**61352 Bad Homburg (DE)**

(72) Erfinder:
• **WEHMEYER, Wolfgang**
**72076 Tübingen (DE)**
• **WÜPPER, Andreas**
**64572 Büttelborn (DE)**

(74) Vertreter: **Ziermann, Oliver**
**Fresenius Medical Care AG & Co. KGaA**
**Global Patents & IP**
**Else-Kröner-Strasse 1**
**61352 Bad Homburg (DE)**

(56) Entgegenhaltungen:
**EP-A1- 0 911 043          WO-A1-2006/072271**
**WO-A1-2008/135193     DE-A1- 4 239 937**
**DE-A1-102008 050 849**

**Beschreibung**

Die Erfindung betrifft eine Vorrichtung zur Erkennung eines Betriebszustandes einer extrakorporalen Blutbehandlung, insbesondere eine Vorrichtung und ein Verfahren zur Erkennung oder Bestimmung einer Ursache einer Abweichung von einem idealen Betriebszustand einer extrakorporalen Blutbehandlung mit einer Blutbehandlungsvorrichtung, bei der das zu behandelnde Blut in einem extrakorporalen Blutkreislauf die Blutkammer eines durch eine semipermeable Membran in die Blutkammer und eine Dialysierflüssigkeitskammer unterteilten Dialysators durchströmt und Dialysierflüssigkeit in einem Dialysierflüssigkeitskreislauf die Dialysierflüssigkeitskammer des Dialysators durchströmt.

Darüber hinaus betrifft die Erfindung ein Verfahren zur Erkennung oder Bestimmung einer Ursache einer Abweichung von einem idealen Betriebszustand oder einem idealen Behandlungsverlauf einer extrakorporalen B1utbehandlung.

**Hintergrund**

In der Nierenersatztherapie sind verschiedene Blutreinigungsverfahren etabliert, bei denen das Blut extrakorporal von harnpflichtigen Blutinhaltsstoffen, das heißt Blutinhaltsstoffen, die bei einem Gesunden über die Nieren ausgeschieden werden, befreit wird. Bei der Hämodialyse findet dazu ein diffusiver Stofftransport von in dem Blut enthaltenen harnpflichtigen Substanzen über eine semipermeable Membran in eine Dialysierflüssigkeit statt. Der Stofftransport findet über die semipermeable Wand eines Dialysators statt, der eine an einen extrakorporalen Blutkreislauf angeschlossene Blutkammer und eine an einen Dialysierflüssigkeitskreislauf angeschlossene Dialysierflüssigkeitskammer aufweist. Blutkammer und Dialysierflüssigkeitskammer sind dabei von der semipermeablen Membran getrennt. Um einen diffusiven Verlust von Elektrolyten, die im Blut verbleiben sollen, zu verhindern, enthält die Dialysierflüssigkeit eine bestimmte Zusammensetzung von Elektrolyten, in einer physiologischen Konzentration.

Demgegenüber findet bei der Hämofiltration ein konvektiver Stofftransport über eine semipermeable Membran eines Filters statt, bei der ein Druckgradient an der Membran die treibende Kraft für den Stofftransport ist. Zum Ausgleich eines Verlustes von erwünschten Blutinhaltsstoffen müssen die über die Membran verlorenen Elektrolyte durch eine Substituatflüssigkeit ersetzt werden. Die Substituatflüssigkeit kann über eine Injektionsstelle stromauf oder stromab des Filters erfolgen. Im ersten Fall spricht man von Prädilution, im zweiten von Postdilution. Eine Kombination eines konvektiven und eines diffusiven Transports wird als Hämodiafiltration bezeichnet. Wenn im Kontext dieser Anmeldung von einer Dialyse oder einer Dialysebehandlung die Rede ist, soll darunter sowohl eine rein diffusive Dialyse oder eine Hämodiafiltration gemeint sein.

Bei der Überwachung einer extrakorporalen Blutbehandlung ist die Überwachung der Blutreinigungsleistung oder Clearance von Bedeutung. Die Clearance K ist definiert, als der Anteil des Blutflusses durch die Blutkammer, die vollständig von Toxinen, insbesondere Urea, gereinigt wird. In der Praxis wird an Stelle der Clearance die Dialysance bestimmt, bei der die Durchgängigkeit der Filtermembran für in der Dialysierflüssigkeit enthaltenen Elektrolyte gemessen wird. Dazu wird die Konzentration eines oder mehrerer Elektrolyte in der Dialysierflüssigkeit im Dialysierflüssigkeitskreislauf stromauf der Dialysierflüssigkeitskammer verändert und die daraus resultierende Änderung der Konzentration stromab der Dialysierflüssigkeitskammer bestimmt. Die geänderte Konzentration der einen oder mehreren Elektrolyte stromauf sowie stromab der Dialysierflüssigkeitskammer beeinflusst die Leitfähigkeit der Dialysierflüssigkeit und wird mittels entsprechender Leitfähigkeitssensoren stromauf und stromab der Dialysierflüssigkeitskammer gemessen. Ein solches Verfahren zur Bestimmung der Clearance während der laufenden Behandlung wird als online Clearance Monitoring bezeichnet und ist in dem Europäischen Patent EP 0 911 043 der Anmelderin offenbart. Ein kommerzielles Verfahren, das auf diesem Prinzip beruht, wird von der Firma Fresenius Medical Care Deutschland GmbH unter der Bezeichnung OCM (Online Clearance Monitor) vertrieben.

Die Dialysance oder Clearance hängt ab von einer Vielzahl von Faktoren, etwa dem Filtertyp, der effektiven Fläche der Filtermembran, der Permeabilität der Filtermembran, der Flussrate des Blutflusses, der Flussrate der Dialysierflüssigkeit. Für sich genommen stellt die Bestimmung der Dialysance oder Clearance daher kein aussagekräftiges Maß für die Erkennung oder Bestimmung einer Betriebssituation oder eines Betriebszustandes einer extrakorporalen Blutbehandlung dar.

Eine Abweichung von einem idealen Betriebszustand der extrakorporalen Blutbehandlung kann dadurch auftreten, dass Blut, das aus dem venösen Zweig des extrakorporalen Blutkreislaufs in den Gefäßzugang des Patienten geleitet wird, von dort unmittelbar in den arteriellen Zweig des extrakorporalen Blutkreislaufs gelangt. Dieses Phänomen wird Fistelrezirkulation genannt, soweit es sich um eine Fistel als Gefäßzugang handelt. Ein entsprechendes Phänomen, bei dem im extrakorporalen Blutkreislauf gereinigtes Blut über den kardiopulmonalen Patientenkreislauf und von dort in den arteriellen Zweig des extrakorporalen Blutkreislaufs geleitet wird, wird als kardiopulmonale Rezirkulation bezeichnet.

Bekannte Verfahren zur Bestimmung der Rezirkulation beruhen auf einer Beeinflussung physikalischer oder chemischer Kenngrössen im venösen Zweig des extrakorporalen Blutkreislaufs und einer sich anschließenden Messung der physikalischen oder chemischen Kenngröße im arteriellen Zweig des extrakorporalen Blutkreislaufs. Beispielsweise kann die Bluttemperatur im venösen Zweig des Blutkreislaufs verändert werden, indem Wärme entweder unmittelbar entzogen wird oder die Temperatur der Dialysierflüssigkeit für eine kurze Zeit abgesenkt wird, wobei sich der dialysatseitige Temperaturabfall über die semi-permeable Membran in einer vorbestimmten Weise auf die Blutseite auswirkt. Zur Messung der Auswirkung der Temperaturabsenkung auf den arteriellen Zweig des Blutkreislaufs kann ein Temperaturaufnehmer im arteriellen Blutkreislauf vorgesehen sein. Im Allgemeinen muss zur Bestimmung der Rezirkulation eine Vorrichtung zur Veränderung einer physikalischen oder chemischen Eigenschaft des im venösen Zweig des extrakorporalen Blutkreislaufs strömenden Blutes sowie ein Messaufnehmer zur Aufnahme einer Messgröße des in den arteriellen Zweig des extrakorporalen Blutkreislaufs zurückströmenden Blutes vorgesehen sein. Diese Maßnahmen bewirken einen erhöhten apparativen Aufwand des Blutbehandlungsgerätes.

In der WO 2006/072271 wird eine Vorrichtung bzw. ein Verfahren offenbart, die auf der Erkenntnis basieren, dass eine Rezirkulation mit einer Veränderung, insbesondere einer Verringerung der Dialyse oder Clearance verbunden ist. Andererseits kann eine Verringerung der Dialysance oder Clearance auch auf andere Komplikationen als die einer Rezirkulation zurückzuführen sein. Bei der in der WO 2006/072271 vorgeschlagenen Vorrichtung bzw. dem Verfahren wird daher nach einer Feststellung einer Veränderung der Dialysance oder Clearacne mit einer zweiten Messung überprüft, ob die Ursache der Veränderung tatsächlich in dem Auftreten einer Rezirkulation liegt. Auch die in der WO 2006/072271 vorgeschlagenen Maßnahmen bewirken einen erhöhten apparativen Aufwand des Blutbehandlungsgerätes.

Es ist daher die Aufgabe der vorliegenden Erfindung eine Vorrichtung und ein Verfahren zur Erkennung oder zur Bestimmung eines Betriebszustandes bei einer extrakorporalen Blutbehandlung zur Verfügung zu stellen, das zumindest eines der genannten Probleme überwindet.

Zusammenfassung

Diese Aufgabe wird durch eine Vorrichtung nach Anspruch 1, sowie ein Computerprogrammprodukt nach Anspruch 12 gelöst. Vorteilhafte Ausführungsformen sind in den abhängigen Ansprüchen wiedergegeben.

Weiterhin wird eine Vorrichtung zur Erkennung einer Ursache einer Abweichung von einem idealen Betriebszustand oder einem idealen Behandlungsverlauf bei einer extrakorporalen Blutbehandlung offenbart. Bei der extrakorporalen Blutbehandlung durchströmt das zu behandelnde Blut in einem extrakorporalen Blutkreislauf eine Blutkammer eines durch eine semipermeable Membran in die Blutkammer und eine Dialysierflüssigkeitskammer unterteilten Dialysators und Dialysierflüssigkeit in einem Dialysierflüssigkeitskreislauf durchströmt die Dialysierflüssigkeitskammer des Dialysators.

Die Vorrichtung zur Erkennung einer Ursache einer Abweichung von einem idealen Betriebszustand und/oder einem idealen Behandlungsverlauf bei einer extrakorporalen Blutbehandlung enthält Mittel zum Verändern einer physikalischen oder chemischen Kenngröße der Dialysierflüssigkeit stromauf der Dialysierflüssigkeitskammer während der Blutbehandlung, Messmittel zum Messen einer physikalischen oder chemischen Kenngröße der Dialysierflüssigkeit stromab der Dialysierflüssgkeitskammer, eine Einrichtung zum Bestimmen eines Wertes der Dialysance oder Clearance aus einer Veränderung der gemessenen physikalischen oder chemischen Kenngröße zu einem bestimmten Zeitpunkt während der Blutbehandlung, eine Einrichtung zum Feststellen einer Abweichung des bestimmten Wertes der Dialysance oder Clearance von einem einen idealen oder typischen oder komplikationsfreien Behandlungsverlauf repräsentierenden Referenzwert der Dialysance oder Clearance, und einer Auswerteeinrichtung zum Erkennen einer Ursache einer Abweichung von einem idealen Betriebszustand in Abhängigkeit von dem bestimmten Zeitpunkt.

Gemäß einem anderen Aspekt wird die Dialysance oder Clearance folgendermaßen mit folgenden Mitteln bestimmt: Mittel zum Verändern einer physikalischen oder chemischen Kenngröße des Blutes stromauf der Blutkammer während der Blutbehandlung, Messmittel zum Messen einer physikalischen oder chemischen Kenngröße des Blutes stromab der Blutkammer, eine Einrichtung zum Bestimmen eines Wertes der Dialysance oder Clearance aus einer Veränderung der gemessenen physikalischen oder chemischen Kenngröße zu einem bestimmten Zeitpunkt während der Blutbehandlung.

Es wird weiterhin ein Verfahren zur Erkennung einer Ursache einer Abweichung von einem idealen Betriebszustand oder einem idealen Behandlungsverlauf bei einer extrakorporalen Blutbehandlung offenbart. Bei der extrakorporalen Blutbehandlung durchströmt das zu behandelnde Blut in einem extrakorporalen Blutkreislauf eine Blutkammer eines durch eine semipermeable Membran in die Blutkammer und eine Dialysierflüssigkeitskammer unterteilten Dialysators und Dialysierflüssigkeit durchströmt in einem Dialysierflüssigkeitskreislauf die Dialysierflüssigkeitskammer des Dialysators. Das Verfahren beinhaltet die folgenden Schritte:

- Verändern einer physikalischen oder chemischen Größe der Dialysierflüssigkeit stromauf der Dialysierflüssigkeitskammer während der Blutbehandlung
- Messen einer physikalischen oder chemischen Kenngröße der Dialysierflüssigkeit stromab der Dialysierflüssgkeitskammer,

- Bestimmen eines Wertes der Dialysance oder Clearance aus einer Veränderung der gemessenen physikalischen oder chemischen Kenngröße zu einem bestimm-ten Zeitpunkt während der Blutbehandlung,
- Feststellen einer Abweichung des bestimmten Wertes der Dialysance oder Clearance von einem einen idealen oder typischen oder Behandlungsverlauf repräsentierenden Referenzwert der Dialysance oder Clearance, und
- Erkennen einer Ursache einer Abweichung von einem idealen Betriebszustand der Blutbehandlung in Abhängigkeit von dem bestimmten Zeitpunkt.

Gemäß einem anderen Aspekt wird die Dialysance oder Clearance folgendermaßen bestimmt:

- Verändern einer physikalischen oder chemischen Größe des Blutes stromauf der Blutkammer während der Blutbehandlung,
- Messen einer physikalischen oder chemischen Kenngröße des Blutes stromab der Blutkammer, und
- Bestimmen eines Wertes der Dialysance oder Clearance aus einer Veränderung der gemessenen physikalischen oder chemischen Kenngröße zu einem bestimm-ten Zeitpunkt während der Blutbehandlung.

Gemäß einem weiteren Aspekt wird eine weitere Vorrichtung zur Erkennung einer Ursache einer Abweichung von einem idealen Betriebszustand oder einem idealen Behandlungsverlauf bei einer extrakorporalen Blutbehandlung bereitgestellt. Gemäß diesem weiteren Aspekt wird ebenfalls von einer extrakorporalen Blutbehandlung ausgegangen, bei der das zu behandelnde Blut in einem extrakorporalen Blutkreislauf eine Blutkammer eines durch eine semipermeable Membran in die Blutkammer und eine Dialysierflüssigkeitskammer unterteilten Dialysators durchströmt und Dialysierflüssigkeit in einem Dialysierflüssigkeitskreislauf die Dialysierflüssigkeitskammer des Dialysators durchströmt.

Die Vorrichtung zur Erkennung einer Ursache einer Abweichung von einem idealen Betriebszustand und/oder einem idealen Behandlungsverlauf bei einer extrakorporalen Blutbehandlung enthält Messmittel zum Messen einer physikalischen oder chemischen Kenngröße der Dialysierflüssigkeit stromab der Dialysierflüssgkeitskammer, wobei die Kenngrösse geeignet ist zur Bestimmung der Konzentration einer Substanz, die in der Dialysierflüssigkeit stromauf der Dialysierflüssigkeitskammer nicht oder nur in einer vernachlässigbaren Konzentration vorhanden ist, wie etwa Harnstoff, eine Einrichtung zum Bestimmen eines Wertes der Konzentration dieser Substanz in der Dialysierflüssigkeit oder im Blut zu einem bestimmten Zeitpunkt während der Blutbehandlung, eine Einrichtung zum Feststellen einer Abweichung des bestimmten Wertes der Konzentration der Substanz von einem einen idealen oder typischen Behandlungsverlauf repräsentierenden Referenzwert der Konzentration oder von einem idealen zeitlichen Konzentrationsprofil während der Behandlung, und einer Auswerteeinrichtung zum Erkennen einer Ursache einer Abweichung von einem idealen Betriebszustand in Abhängigkeit von dem bestimmten Zeitpunkt.

Im Einklang mit dem weiteren Aspekt wird ein Verfahren zur Erkennung einer Ursache einer Abweichung von einem idealen Betriebszustand oder einem idealen Behandlungsverlauf bereitgestellt. Bei der extrakorporalen Blutbehandlung durchströmt das zu behandelnde Blut in einem extrakorporalen Blutkreislauf eine Blutkammer eines durch eine semipermeable Membran in die Blutkammer und eine Dialysierflüssigkeitskammer unterteilten Dialysators und Dialysieflüssigkeit durchströmt in einem Dialysierflüssigkeitskreislauf die Dialysierflüssigkeitskammer des Dialysators.

Das Verfahren beinhaltet die folgenden Schritte:

- Messen einer physikalischen oder chemischen Kenngröße der Dialy-sierflüssigkeit stromab der Dialysierflüssgkeitskammer, wobei die Kenngrösse geeignet ist zur Bestimmung der Konzentration einer Substanz, die in der Dialysierflüssigkeit stromauf der Dialysierflüssigkeitskammer nicht oder nur in einer vernachlässigbaren Konzentration vorhanden ist, wie etwa Harnstoff,
- Bestimmen eines Wertes der Konzentration der Substanz in der Dialysierflüssig-keit oder im Blut zu einem bestimmten Zeitpunkt während der Blutbehandlung,
- Feststellen einer Abweichung des bestimmten Wertes der Konzentration der Substanz von einem einen idealen oder typischen Behandlungsverlauf repräsentierenden Referenzwert der Konzentration oder von einem idealen zeitlichen Konzentrationsprofil während der Behandlung, und
- Erkennen einer Ursache einer Abweichung von einem idealen Betriebszustand in Abhängigkeit von dem bestimmten Zeitpunkt.

Kurzbeschreibung der Zeichnungen
Weitere Einzelheiten und Vorteile der Erfindung werden anhand eines in der Zeichnung dargestellten Ausführungsbeispiels näher beschrieben. Es zeigen

Figur 1 ein Blockschaltbild eines Dialysators mit einer Vorrichtung zur Erkennung der Ursache einer Abweichung von einem idealen Betriebszustand,

Figur 2a ein Schaubild eines Dialysators mit Zu- und Ablauf und in den mathematischen Herleitungen verwendeten Symbole,

Figur 2b den zeitlichen Verlauf der gemessenen Elektrolytkonzentration stromauf und stromab des Dialysators nach einem Konzentrationsbolus,

Figur 3 den zeitlichen Verlauf einer gemessenen Dialysance und den zeitlichen Verlauf der Dialysance bei einem idealen Behandlungsverlauf,

Figur 4 ein Flussdiagramm eines Verfahren zur Erkennung einer Ursache einer Abweichung von einem idealen Betriebszustand bei einer extrakorporalen Blutbehandlung,

Figur 5 ein Flussdiagramm eines Verfahrens zum Erkennen einer Ursache einer Abweichung von einem idealen Betriebszustand der Blutbehandlung in Abhängigkeit von dem bestimmten Zeitpunkt, und

Figur 6 ein Blockdiagramm einer Netzwerks von Dialysemaschinen zur Durchfüh-rung eines Verfahrens zur Erkennung der Ursache einer Abweichung von einem idealen Betriebszustand.

Detaillierte Beschreibung der Zeichnungen

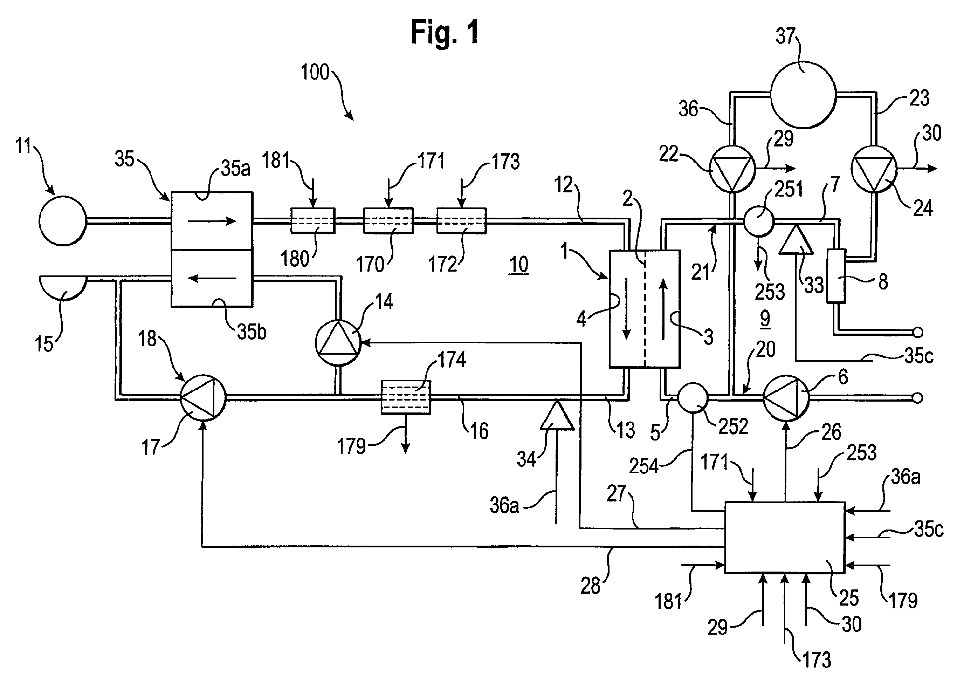

Figur 1 zeigt eine Dialysevorrichtung 100 mit einer Steuer- und Kontrolleinheit 25 zur Erkennung einer Ursache einer Abweichung von einem idealen Betriebszustand oder einem idealen Behandlungsverlauf bei einer extrakorporalen Blutbehandlung.

Die Dialysevorrichtung 100 besteht im Wesentlichen aus einem Blutkreislauf 9, einem Dialysierflüssigkeitskreislauf 10 sowie aus einem Dialysator 1 der von einer Membran 2 in eine Blutkammer 3 des Blutkreislaufs 9 und eine Dialysierflüssigkeitskammer 4 des Dialysierflüssigkeitskreislaufs 10 geteilt wird. Der extrakorporale Blutkreislauf 9 besteht aus einem arteriellen Zweig 20 mit einer arteriellen Blutleitung 5, der von dem Patienten entnommenes Blut zu der Blutkammer 3 hin führt. Der arterielle Zweig 20 enthält typischerweise eine Blutpumpe 6, die beispielsweise als Rollenpumpe ausgeführt sein kann und die über eine Steuerleitung 26 von der Steuer- und Kontrollvorrichtung 25 ansteuerbar ist. In der Blutkammer 3 gereinigtes Blut wird über eine venöse Leitung 7 eines venösen Zweiges 21 an den Patienten zurückgegeben. Die venöse Leitung enthält typischerweise eine Tropfkammer 8 zur Entgasung des Blutes, bevor es dem Patienten zurückgegeben wird.

Im Falle einer Hämodiafiltrationsbehandlung ist der extrakorporale Blutkreislauf mit einer Quelle für Substituatflüssigkeit 37 verbunden, von der aus Substituatflüssigkeit mittels einer venösen Substituatpumpe 24 über eine venöse Substituatleitung 23 in die Tropfkammer 8 zugegeben werden kann (Postdilution). Dazu kann die venöse Substituatpumpe 24 über eine Steuerleitung 30 mit der Steuereinheit 25 verbunden sein. Zusätzlich oder alternativ dazu kann eine arterielle Substituatleitung 36 vorgesehen sein, durch die mittels einer arteriellen Substituatpumpe 22 dem arteriellen Blutkreislauf 20 Substituatflüssigkeit zugegeben werden kann (Prädilution). Um dies zu kontrollieren, kann die arterielle Substituatpumpe 22 über eine Steuerleitung 29 mit der Steuereinheit 25 verbunden sein.

Der Dialysierflüssigkeitskreislauf enthält eine mit einer Dialysierflüssigkeitsquelle 11 verbundene Dialysierflüssigkeitszuführleitung 12, die die Dialysierflüssigkeit zu der Dialysierflüssigkeitskammer 4 des Dialysators 1 bringt. Verbrauchte Dialysierflüssigkeit wird über eine Dialysierflüssigkeitsabführleitung mit Abschnitten 13, 16 in einen Abfluss 15 entsorgt. Die Dialysierflüssigkeit im Dialysierflüssigkeitskreislauf wird durch eine Dialysierflüssigkeitspumpe 14 umgewälzt. Eine Bilanzkammerpumpe 35 bilanziert zwischen der durch die erste Bilanzkammer 35a geförderten frischen Dialysierflüssigkeit und der durch die zweite Bilanzkammer 35b fließenden verbrauchten Dialysierflüssigkeit. Im Falle der Hämodilafiltration pumpt eine Ultrafiltrationspumpe 17 verbrauchte Dialysierflüssigkeiten an der Bilanzkammer vorbei. Die von der Diafiltrationspumpe abgepumpte Flüssigkeitsmenge kann als Maß für die durch die Membran 2 abgezogene Flüssigkeit herangezogen werden. Die Dialysierflüssigkeitspumpe 14 ist von der Steuer- und Kontrollvorrichtung 25 über eine Steuerleitung 27 ansteuerbar und die Ultrafiltrationspumpe 17 ist von der Steuer- und Kontrollvorrichtung 25 über eine Steuerleitung 28 ansteuerbar.

Die Dialysierflüssigkeitszuführleitung enthält ein Boluserzeugungsmittel 170 mit dessen Hilfe ein Konzentrationsbolus zum Beispiel eines Elektrolyts erzeugt werden kann, dessen Dialysance bestimmt werden soll. Das Boluserzeugungsmittel 170 ist über eine Datenleitung 171 mit der Kontroll- und Auswerteeinrichtung 25 verbunden, die über die Datenleitung 171 das Boluserzeugungsmittel so ansteuern kann, dass ein Konzentrationsbolus erzeugt wird. Der Konzentrationsbolus kann ein positiver Konzentrationsbolus sein, bei dem eine vorbestimmte Menge eines Konzentrats, etwa eines Elektrolyts, in die Dialysierflüssigkeitszuführleitung zugegeben wird oder aber ein negativer Konzentrationsbolus in der Dialysierflüssigkeit, bei der eine vorbestimmte Menge einer Verdünnungsflüssigkeit, wie etwa destilliertes Wasser der Dialysierflüssigkeit zugegeben wird. Das Boluserzeugungsmittel kann so ausgebildet sein, eine Vielzahl von Flüssigkeitsboli zu aufeinander folgenden Zeitpunkten zu erzeugen. Das Konzentrationsprofil des Flüssigkeitsbolus kann entweder in einem Kalibrierungsschritt vor der eigentlichen Anwendung des Konzentrationsbolus bestimmt werden, wenn für eine ausreichende Reproduzierbarkeit des Konzentrationsprofils gesorgt werden kann. Als Alternative kann das Konzentrationsprofil mit einem stromauf der Dialysierflüssigkeitskammer und stromab des Boluserzeugungsmittels 170 in der Zuführleitung der Dialysierflüssigkeit angeordneten Konzentrationsmessfühler 172 während der Gabe des Konzentrationsbolus gemessen werden. Als Konzentrationsmessfühler 172 kann beispielsweise ein Leitfähigkeitsmesser verwendet werden. Der Konzentrationsmessfühler 172 ist durch eine Datenleitung 173 mit der Kontroll- und Auswerteeinheit 25 verbunden, die den Verlauf der Boluskonzentration stromauf des Dialysators während der Bolusgabe in einer entsprechenden Speichereinheit der Kontroll- und Auswerteeinheit 25 aufzeichnen kann. Alternativ kann ein zuvor in einem Kalibrierungsschritt gewonnenes Maß für den Konzentrationsbolus in einer Speichereinheit der Kontroll- und Auswerteeinheit 25 abgelegt sein.

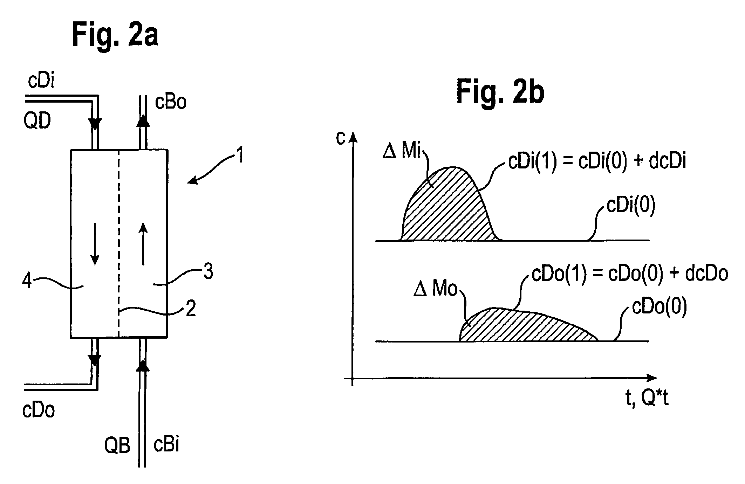

Wie in Figur 2b genauer gezeigt ist, verändert sich das Profil des Konzentrationsbolus während der Konzentrationsbolus durch die Flüssigkeitskammer 4 des Dialysators 1 geleitet wird. Der Konzentrationsbolus der durch die Flüssigkeitskammer 4 geleiteten Dialysierflüssigkeit kann mit dem stromab der Dialysierflüssigkeitskammer 4 in der Abflussleitung der Dialysierflüssigkeit angeordneten Konzentrationsmessfühler 174 gemessen werden. Der Konzentrationsmessfühler 174 kann als Leitfähigkeitssensor ausgebildet sei und ist über eine Datenleitung 179 mit der Kontroll- und Auswerteeinheit 25 verbunden, die den Verlauf der Boluskonzentration nach der Bolusgabe stromab des Dialysators in einer entsprechenden Speichereinheit aufzeichnen kann. Aus dem Vergleich der Boluskonzentration stromauf des Dialysators und der Boluskonzentration stromab des Dialysators kann die Kontroll- und Auswerteeinheit 25 unter Hinzuziehung des unten in Verbindung mit den Figuren 2a und 2b erläuterten Formalismus die Dialysance des Konzentrats oder Elektrolyts und damit indirekt auch die Clearance bestimmen.

In einer vorteilhaften Ausführung umfasst die Dialysevorrichtung 1 Messmittel zur Bestimmung der Rezirkulation, die auf einem Temperaturbolus beruhen. Unter Rezirkulation soll hier der Wiedereintritt von durch die venöse Leitung in den Patienten zurückgegebenem Blut in die arterielle Leitung verstanden werden. Ein solches Messmittel zur Bestimmung der Rezirkulation umfasst typischerweise ein Boluserzeugungsmittel 180 zum Erzeugen eines Temperaturbolus in der Dialysierflüssigkeitszuführleitung 12, das über eine Steuerleitung 181 mit der Kontroll- und Auswerteeinheit 25 verbunden ist. Der Temperaturbolus in der Dialysierflüssigkeitszuführleitung wird durch den Dialysator 1 auf den Blutkreislauf 9 übertragen. Dort ist er mit einem Temperatursensor 251 messbar, der über eine Datenleitung 253 mit der Kontroll- und Auswerteeinheit 25 verbunden ist. Der Temperaturbolus in der venösen Blutleitung 21 breitet sich entlang der Rezirkulationswege im Körper des Patienten aus und ist dann in der arteriellen Blutleitung 20 über einen arteriellen Temperatursensor 252 messbar. Messergebnisse des arteriellen Temperatursensors 252 sind über die Datenleitung 254 an die Kontroll- und Auswerteeinheit 25 übertragbar.

Durch einen Vergleich des arteriell gemessenen und des venös gemessenen Temperaturbolus kann auf die Rezirkulation des über die venöse Leitung 7 zurückgegebenen Blutes zurück in die arterielle Seite geschlossen werden. Ein entsprechendes Verfahren ist in der WO 2006/072271 offenbart und wird ausdrücklich in den Offenbarungsgehalt der vorliegenden Anmeldung aufgenommen. In der angesprochenen bevorzugten Ausführungsform ist die Kontroll- und Auswerteeinheit 25 zur Durchführung dieses Messverfahrens konfiguriert.

In einer weiteren bevorzugten Ausführungsform enthält die Dialysevorrichtung 1 Messmittel zur Bestimmung eines Clottings der semipermeablen Membran. Dazu enthält die Dialysevorrichtung 1 einen ersten Schallaufnehmer 34 im Dilaysierflüssigkeitskreislauf und einen zweiten Schallaufnehmer 33 im extrakorporalen Blutkreislauf. Der erste und der zweite Schallaufnehmer sind geeignet zur Aufnahme eines Druckpulses, der von einem Druckpulserzeugungsmittel im Dialysierflüssigkeitskreislauf oder im Blutkreislauf erzeugt wird. Ein Druckpulserzeugungsmittel im Blutkreislauf wäre etwa die Blutpumpe 6, ein Druckpulserzeugungsmittel im Dialysierflüsigkeitskreislauf wäre etwa die Dialysierflüssigkeitspumpe 14 oder die Ultrafiltrationspumpe 17.

Durch einen Vergleich der von dem ersten und dem zweiten Schallaufnehmer 34 und 33 aufgenommenen Druckpulse ist ein Rückschluss auf die Dämpfung von Schallwellen im Dialysator und dadurch ein Rückschluss auf die Schalldämpfung durch die Dialysatormembran möglich. Diese erlaubt wiederum einen Rückschluss auf den Strömungswiderstand der Membran, den Zustand der Membranporen und ein mögliches Clotting der Membran.

Ein solches Verfahren zur Bestimmung des Zustandes der Dialysatormembran durch den Vergleich von Druckmessungen im Dialysierflüssigkeitszweig und im extrakorporalen Blutkreislauf ist in der WO 2008/135193 offenbart und wird ausdrücklich in den Offenbarungsgehalt der vorliegenden Anmeldung aufgenommen.

Die Drucksensoren 34 und 33 sind über Datenleitungen 36a beziehungsweise 35c mit der Kontroll- und Auswerteeinheit 25 verbunden, die die entsprechenden Auswertungen zur Bestimmung der Schalldämpfung im Dialysator und zum Bestimmen eines Clottings der Membranporen vornimmt.

Als Druckpulserzeugungsmittel besonders geeignet ist die Dialysierflüssigkeitspumpe 14, wenn Sie als Okklusionspumpe ausgelegt ist. In diesem Fall erzeugt Sie eine Serie von Druckpulsen oder oszilierenden Pulsen. Diese oszilierenden Pulse können auf der Seite des extrakorporalen Blutkreislaufs mittels des Schallaufnehmers 33 aufgenommen, über die Datenleitung 35c zu der Kontroll- und Auswerteeinheit 25 übertragen und dort ausgewertet werden.

Die von der Blutpumpe 6 erzeugten Druckpulse werden über die arterielle Blutleitung auf die Blutkammer 3 des Dialysators 1 übertragen und von dort über die Membran 2 des Dialysators 1 auf den Dialysierflüssigkeitskreislauf. Dort können Sie mit dem Schallaufnehmer 34 aufgenommen, über die Datenleitung 36a zu der Kontroll- und Auswerteeinheit 25 übertragen und darin ausgewertet werden.

Diese Auswertung kann beispielsweise dadurch erfolgen, dass das von dem Drucksensor 33 aufgenommene Signal und das von dem Sensor 34 aufgenommene Signal jeweils einer Spektralanalyse unterzogen werden, und die Spektren des mit dem Drucksensor 33 aufgenommenen Signals und des mit dem Drucksensor 34 aufgenommenen Signals miteinander verglichen werden. Dazu kann die Kontroll- und Auswerteeinheit etwa eine Fourieranalysevorrichtung enthalten, die das Signal von dem Drucksensor 33 und das Signal von dem Drucksensor 34 in seine Spektralkomponenten zerlegt. Ein Vergleich der Amplitudenspektren des Signals Drucksensors 33 und des Signals des Drucksensors 34 erlaubt einen Rückschluss auf die frequenzabhängige Dämpfung der oszilierenden Pulsen durch die Dialysatormembran 2. Liegt diese über einem Schwellwert, der experimentell bestimmt werden kann, so lässt dies einen Schluss zu, dass die Dialysatormembran mit einer Sekundärmembran belegt ist, das es also zu einem so genannten Clotting gekommen ist.

Figur 2a zeigt einen Dialysator 1 mit einer Dialysierflüssigkeitskammer 4 eines Dialysierflüssigkeitskreislaufs, die von einer Membran 2 von der Blutkammer 3 eines Blutkreislaufs getrennt ist. Der Fluss der Dialysierflüssigkeit wird mit dem Formelzeichen QD und der Blutfluss mit dem Formelzeichen QB bezeichnet. cBi ist die Konzentration des betrachteten Konzentrats etwa eines bestimmten Elektrolyts an Bluteingang, cBo ist die entsprechende Konzentration am Blutausgang. cDi ist die Konzentration des Konzentrats, etwa eines bestimmten Elektrolyts, in der Dialysierflüssigkeit am Dialysateingang und cDo ist die entsprechende Konzentration in der Dialysierflüssigkeit am Dialysatausgang.

Figur 2b zeigt den zeitlichen Verlauf der Veränderung der Dialysierflüssigkeitseingangs- und Ausgangskonzentrationen cDi und cDo. Deutlich ist zu erkennen, dass der Konzentratbolus mit einer zeitlichen Verzögerung am Ausgang des Dialysators auftritt. Die Amplitude des Konzentratbolus ist am Ausgang des Dialysators geringer als am Eingang des Dialysators. Dabei ist nur der Teil des Konzentratbolus zu berücksichtigen, der nicht einer Basis- oder Grundkonzentration zuzuschreiben ist. Die Basis- oder Grundkonzentration auf der Eingangsseite des Dialysators ist dabei mit cDi(0) bezeichnet, die Basis- oder Grundkonzentration auf der Ausgangsseite des Dialysators mit cDo(0). Die Gesamtkonzentration des Konzentrats auf der Eingangsseite des Dialysators ist mit cDi(1) bezeichnet, die Gesamtkonzentration des Konzentrats auf der Ausgangsseite des Dialysators mit cDo(1). Der zu berücksichtigende Teil des Konzentratbolus, d.h. der Anteil der nicht einer Basis- oder Grundkonzentration zuzuschreiben ist, ist mit dcDi für die Eingangsseite des Dialysators und mit dcDo für die Ausgangsseite des Dialysators bezeichnet. Zur Ermittlung der Basiskonzentration können Messwerte vor und/ oder nach dem Bolus herangezogen werden. Die Kontroll- und Auswerteeinheit 190 verfügt über eine Recheneinheit, die aus dem zeitlichen Verlauf der Dialysierflüssigkeitskonzentration stromauf oder stromab des Dialysators und der Dialysierflüssigkeitsrate QD die beiden Größen $\Delta Mi$ und $\Delta Mo$ und nach den folgenden Gleichungen berechnet

$$\Delta Mi = QD * \int dcDi \, dt \qquad (1)$$

$$\Delta Mo = QD * \int dcDo \, dt \qquad (2)$$

Die Dialysance D wird aus den Größen $\Delta Mi$ und $\Delta Mo$ und nach der folgenden Gleichung berechnet:

$$D = QD * \frac{\Delta Mi - \Delta Mo}{\Delta Mi} \qquad (3).$$

Während der Dialysebehandlung führt die Die Kontroll- und Auswerteeinheit 190 fortlaufend zu bestimmten Zeitpunkten Messungen zur Bestimmung der Dialysance oder Clearance durch, beispielsweise in einem Zeitabstand von 20 - 30 Minuten.

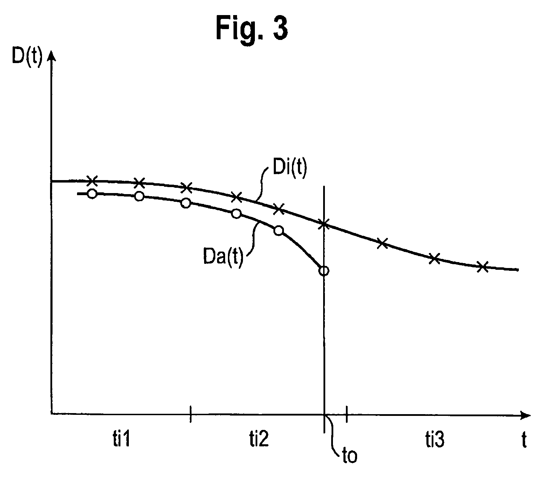

Figur 3 zeigt den zeitabhängigen Verlauf der Dialysance Di(t) in Relation zu einem Startzeitpunkt der Dialysebehandlung bei einem idealen oder komplikationsfreien Verlauf der Dialysebehandlung, dem idealen Verlauf der Dialysance. Der ideale zeitliche Ablauf kann sich durch eine Mittelwertbildung aus komplikationsfreien vorangegangenen Behandlungen ergeben oder durch eine Mittelwertbildung aus komplikationsfreien vergangenen Behandlungen, korrigiert oder kompensiert um einen Faktor oder Anteil, der die Behandlungspara-meter der laufenden Behandlung berücksichtigt. Solche Parameter der laufenden Behandlung können z.B. die momentane Blutflussrate, die momentane Dialysierflüssigkeitsflussrate, die momentane Ultrafiltrationsrate, die Rate der Substituatflüssigkeit, Stelle, an der Substituatflüssigkeit zugegeben wird, d.h. Prädilution oder Postdilution, der Typ des Dialysators, oder der Anschluss der Konnektoren zum Gleich- oder Gegenstrombetrieb sein.

So kann zum Beispiel ausgehend von den bekannten Behandlungsparametern eine theoretische Abschätzung der Clearance angegeben werden, und der zeitabhängige Verlauf der Dialysance kann auf diese theoretische Abschätzung der Clearance normiert werden.

Die Veränderung der Dialysance während der Dialysebehandlung gemäß dem dargestellten idealen zeitlichen Verlauf repräsentiert dabei reproduzierbare Effekte, die bei jeder Dialysebehandlung eines bestimmten Patienten im Wesentlichen gleich verlaufen, wie etwa der Alterung des Pumpensegments, Ablagerungen auf und Verunreinigung der Membran, der Änderung des Wassergehalts des Blutes auf Grund von Ultrafiltration und einem eingeschränkten Refilling, sowie kardiopulmonaler Rezirkulation. So geht die Alterung des Pumpensegments auf die durch die Rollenpumpe ausgeübten Kräfte zurück. Ablagerungen auf der Membran können Proteinablagerungen oder die Bildung einer zweiten Schicht (sogenannte second layer deposition) sein. Die Erhöhung des Hämatokrits und des totalen Proteingehalts des Blutes führt zu einer Reduzierung des Blutwassergehaltes und zu einer Reduzierung der gemessenen Dialysance oder Clearance. Die Erhöhung des Hämatokrits führt auch zu einem reduzierten Blutdruck, der zu einer verminderten Förderleistung des Herzens führt. Dies wiederum führt zu einem Anstieg der kardiopulmonalen Rezirkulation.

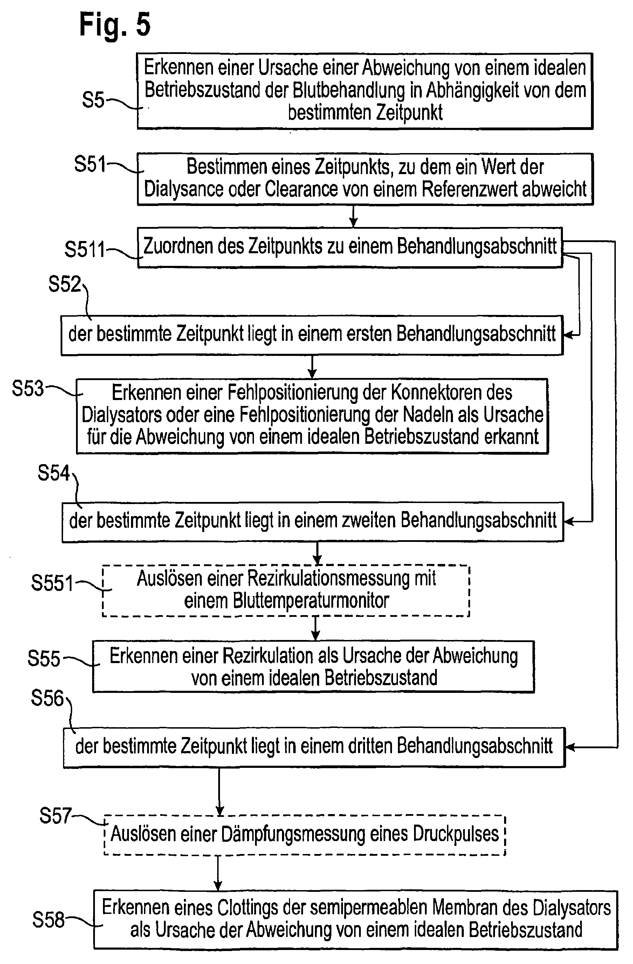

Da(t) sind während einer laufenden Dialysebehandlung zu unterschiedlichen Zeitpunkten nach dem Beginn der Dialysebehandlung bestimmte Messwerte der Dialysance oder Clearance. Zu einem Zeitpunkt to wird eine die aktuell gemessene Dialysance oder Clearance mit dem idealen Verlauf der Dialysance verglichen. Ergibt sich eine signifikante Abweichung, so wird eine Zuordnung des Zeitpunkts to, zu dem die signifikante Abweichung festgestellt wird, zu einem aktuellen Behandlungsabschnitt vorgenommen. Dazu ist die Zeitachse in verschiedene Behandlungsabschnitte eingeteilt, hier einen ersten, zweiten und einen dritten Behandlungsabschnitt ti1, ti2 und ti3. Gezeigt ist eine Zuordnung des Zeitpunkts to zu dem zweiten Behandlungsabschnitt ti2. Die Bedeutung des ersten, zweiten und dritten Behandlungsabschnitts wird unten in Zusammenhang mit der Beschreibung von Figur 5 genauer erläutert.

Wenn der ideale oder komplikationsfreie Verlauf der Dialysance Di(t) bei Behandlungsparametern bestimmt wurde, die von den Behandlungsparametern der laufenden Behandlung abweichen, kann der ideale oder komplikationsfreie Verlauf der Dialysance Di(t) mit dem Wert Da(t) während einer laufenden Dialysebehandlung verglichen werden, wenn sowohl Di(t) als auch Da(t) auf einen theoretischen Wert der Clearance normiert werden, der die jeweiligen Behandlungsparameter berücksichtigt.

So kann etwa für ausgehend von dem Massentransferkoeffizienten KoA, der Flussrate des Dialysats in den Dialysator $Q_{Di}$ und der Blutwasserflussrate in den Dialysator $Q_{Bi}$ im Falle der Hämofiltration folgende Beziehung für eine theoretische Abschätzung oder einen theoretischen Wert $K_{HD}$ der Clearance angegeben werden:

$$K_{HD} = Q_{bw} * \frac{\left[ \exp\left( KoA * \left( \frac{1}{Q_{Bi}} - \frac{1}{Q_{Di}} \right) \right) \right] - 1}{\exp\left( KoA * \left( \frac{1}{Q_{Bi}} - \frac{1}{Q_{Di}} \right) - \frac{Q_{Bi}}{Q_{Di}} \right)} \qquad \text{(Gleichung 1)}$$

Im Fall der Hämodialfiltration müssen zusätzlich die Dialysatflussrate aus dem Dilalysator $Q_{Do}$, die Blutwasserflussrate aus dem Dialysator, und die Rate der Menge des über die Dialysatormembran entzogenen Ultrafiltrats $Q_F$ für die theoretische Abschätzung oder den theoretischen Wert der Clearance $K_{HDF}$ berücksichtigt werden. Legt man die folgenden Hilfsgrößen fest:

$$f = \frac{KoA}{Q_F} - \frac{1}{e^{\frac{Q_F}{KoA}} - 1} \qquad \text{(Gleichung 2)}$$

$$p = KoA + (1 - f) * Q_F \qquad \text{(Gleichung 3)}$$

sowie
$$Z = \left(1 - \frac{Q_F}{Q_{Bi}}\right)^{\frac{p}{Q_F}-1} * \left(1 - \frac{Q_F}{Q_{Do}}\right)^{-\left(\frac{p}{Q_F}-1\right)}$$
(Gleichung 4)

so kann folgende theoretische Abschätzung oder folgender theoretischer Wert für die Clearance $K_{HDF}$ im Fall der Ultrafiltration angegeben werden:

$$K_{HDF} = Q_{Bi} * \frac{1 - \frac{Q_{Do}}{Q_{Di}} * \frac{Q_{Bo}}{Q_{Bi}} * Z}{1 - \frac{Q_{Bo}}{Q_{Di}} * Z}$$
(Gleichung 5)

Mit Hilfe einer theoretischen Abschätzung oder einem theoretischen Wert für die Clearnce bei der Hämodilayse $K_{HD}$ oder der Clearance bei der Hämofiltration $K_{HDF}$ kann der ideale oder komplikationsfreie Verlauf der Dialysance Di(t) als auch der während der laufenden Behandlung gemessene Verlauf der Dialysance Da(t) auf die theoretische Clearance $K_{HD}$ beziehungsweise $K_{HDF}$ normiert werden.

Die Bestimmung, ob eine signifikante Abweichung der während der laufenden Behandlung gemessenen Dialysance Da(t) vorliegt, kann etwa auf folgende Weise erfolgen. Wenn die Behandlungsparameter der aktuellen Behandlung von vergangenen, komplikationsfreien Behandlungen abweichen, so berechnet man zunächst den normierten Wert der Abweichung $\frac{D_a(t) - K_{HD}}{K_{HD}}$ (für die Hämodialyse) oder $\frac{D_a(t) - K_{HDF}}{K_{HDF}}$ (für die Hämofiltration) zwischen der theoretischen Clearance und der gemessenen Dialysance oder Clearance für die ideale, komplikationsfreie Behandlung. Als weiteren Schritt berechnet man für die aktuelle Behandlung die normierte Abweichung zwischen der theoretischen Clearance und der gemessenen Dialysance $\frac{D_i(t) - K_{HD}}{K_{HD}}$ oder $\frac{D_i(t) - K_{HDF}}{K_{HDF}}$. Wenn sich die aktuelle Abweichung signifikant von der idealen, komplikationsfreien unterscheidet, z.B. um mehr als 10 Prozent (z.B.

$$\left| \frac{D_i(t) - K_{HD}}{K_{HD}} \right| = 5\% \qquad , \qquad \left| \frac{D_a(t) - K_{HD}}{K_{HD}} \right| \geq 15\% \qquad ,$$ so wird daraus auf das Vorliegen einer Störung geschlossen.

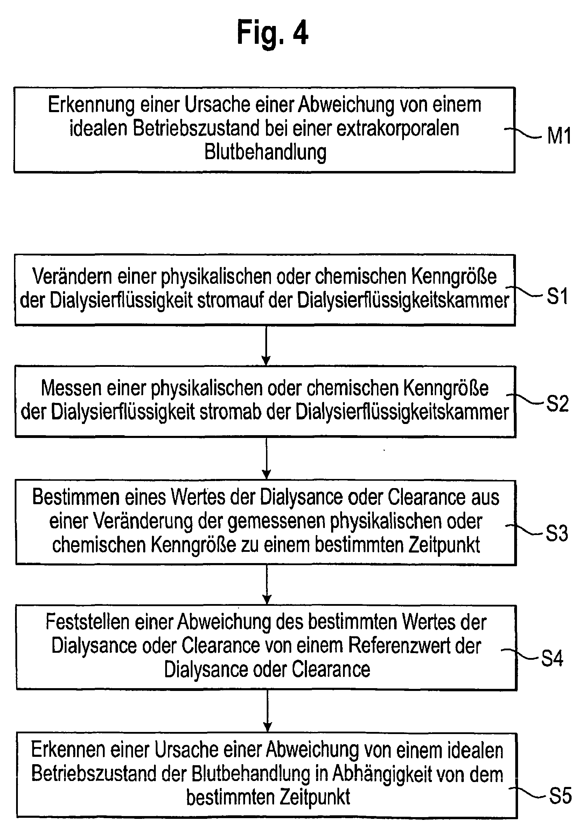

Figur 4 zeigt eine Verfahren M1 zur Erkennung einer Abweichung von einem idealen Betriebszustand bei einer extrakorporalen Blutbehandlung. Der ideale Betriebszustand entspricht dabei einem Verlauf der Behandlung in dem keine Komplikationen oder besonderen Ereignisse auftreten.

In einem Schritt S1 wird eine physikalische oder chemische Kenngröße der Dialysierflüssigkeit stromauf der Dialysierflüssigkeitskammer verändert. Die physikalische oder chemische Größe kann etwa eine Elektrolykonzentration in der Dialysierflüssigkeit stromauf der Dialysierflüssigkeitskammer sein. Diese Elektrolytkonzentration kann durch eine Zugabe einer vorbestimmten Menge eines Konzentrats oder Elektrolyts in die Dialysierflüssigkeit oder aus einer Zubereitung der Dialysierflüssigkeit unter Verwendung einer vorbestimmten Menge eines Elektrolyts ergeben. Der so erzeugte Konzentrationsbolus kann ausgemessen werden, indem die Elektrolytkonzentration stromauf der Dialysierflüssigkeitskammer mittels einer Leitfähigkeitsmessung in ihrem zeitlichen Verlauf bestimmt wird, wenn für das bestimmte Elektrolyt das Verhältnis zwischen der Konzentration des Elektrolyts und der Leitfähigkeit und gegebenenfalls die Konzentration weiterer Elektrolyte vorbekannt ist.

In einem zweiten Schritt S2 wird die physikalische oder chemische Kenngrösse der Dialysierflüssigkeit stromab der Dialysierflüssigkeitskammer in ihrem zeitlichen Verlauf bestimmt, um ein Konzentrationsprofil stromab des Dialysators zu bestimmen. Im Falle der Elektrolytkonzentration als physikalischer oder chemischer Kenngröße kann dies mit Hilfe einer stromab des Dialysators im Dialysierflüssigkeitskreislauf angeordnete Leitfähigkeitszelle geschehen.

In einem dritten Schritt S3 wird aus der Veränderung der gemessenen physikalischen Größe zu einem bestimmten Zeitpunkt die Dialysance oder Clearance bestimmt. Zum Beispiel kann aus dem Konzentrationsbolus und dem zeitlichen Verlauf der physikalischen oder chemischen Kenngröße die Dialysance oder Clearance bestimmt werden. Dies kann etwa geschehen indem das Bolusprofil und das stromab des Dialysators gemessene Konzentrationsprofil unter Verwendung des Formalismus der Figuren 2a und 2b mit einander verglichen werden. Der Zeitpunkt, zu dem die Dialysance oder Clearance bestimmt wird, kann dabei ein beliebiger, vorbestimmter Zeitpunkt innerhalb des Messintervals sein.

Anschließend wird in einem vierten Schritt S4 eine Abweichung des bestimmten Wertes der Dialysance oder Clearance von einem vorbestimmten Referenzwert der Dialysance oder Clearance bestimmt, der einen idealen oder komplikationsfreien Behandlungsverlauf repräsentiert.

Dabei kann der vierte Schritt einen Bestimmungsschritt enthalten, ob es sich bei der festgestellten Abweichung um eine signifikante Abweichung handelt. Die Abweichung kann dabei so sein, dass die gemessene Dialysance oder Clearance höher oder niedriger als die einem idealen Behandlungsverlauf entsprechende Dialysance oder Clearance ist. Ob die Abweichung eine signifikante Abweichung darstellt, kann durch den Vergleich des festgestellten Unterschiedes zwischen gemessener und idealer Clearance oder Dialysance mit einem vorbestimmten Schwellwert erfolgen oder als eine relative, prozentuale oder normierte Abweichung ergeben. Liegt die Abweichung betragsmäßig unter dem vorbestimmten Schwellwert, so wird eine vorhandene Abweichung als eine nicht signifikante Abweichung klassifiziert. Liegt die Abweichung betragsmäßig über dem vorbestimmten Schwellwert, so wird eine vorhandene Abweichung als eine signifikante Abweichung klassifiziert. Eine signifikante Abweichung kann etwa eine Abweichung mehr als 10% über oder unter dem vorbestimmten Schwellwert oder mehr als 15% über oder unter dem vorbestimmten Schwellwert sein. Die Klassifizierung einer Dialysance oder Clearance in Abhängigkeit von einer prozentual angegebenen Abweichung als signifikant kann dabei so erfolgen, dass der Prozentsatz bei dem eine Abweichung signifikant ist, in Abhängigkeit von dem Behandlungsabschnitt vorgenommen wird.

Ist die Abweichung signifikant, so kann an der Dialysemaschine eine Fehlermeldung angezeigt werden, die eine signifikante Abweichung der Dialysance oder Clearance meldet. Alternativ oder zusätzlich kann eine signifikante Abweichung in einem Patientendatensatz, etwa in einer Patientendatenbank festgehalten werden.

Die Feststellung, ob eine signifikante Abweichung von einem Referenzwert vorliegt, kann dabei in einer Weise erfolgen, dass eine relative Abweichung während des Behandlungsverlaufs bestimmt wird. Dies kann in der Weise geschehen, dass eine zuvor bestimmte Abweichung von einem Referenzwert "herausgerechnet" wird, und für die Bestimmung, ob eine signifikante Abweichung vorliegt, nur noch eine weitere Abweichung gegenüber einer bereits zuvor bestimmten Abweichung berücksichtigt wird.

Ist die Abweichung signifikant, so wird in einem sechsten Schritt S5 die Ursache einer Abweichung in Abhängigkeit von dem bestimmten Zeitpunkt erkannt. Dazu kann etwa der Zeitpunkt, zu dem zuvor die Dialysance oder Clearance bestimmt wurde, und damit auch der Zeitpunkt, zu dem die Abweichung der Dialysance oder Clearance von dem idealen zeitlichen Verlauf bestimmt wurde, einem bestimmten Behandlungsabschnitt zugeordnet werden, und in Abhängigkeit von dem bestimmten Behandlungsabschnitt kann die Ursache der Abweichung von einem idealen Behandlungsverlauf erkannt werden. Mit anderen Worten: die Ursache einer Abweichung von einem idealen Behandlungsverlauf kann einem bestimmten Behandlungsabschnitt zugeordnet werden. Die Zuordnung kann dabei auch in einer unscharfen Weise erfolgen, etwa in der Art, dass Wahrscheinlichkeiten für die entsprechenden Ursachen angegeben werden. Unter der Ursache einer Abweichung von einem idealen Behandlungsverlauf oder einem idealen Betriebszustand des Dialysators soll auch eine bestimmte Gruppe von möglichen Ursachen verstanden werden, wobei die Zuordnung in diesem Fall zu einer Gruppe von Ursachen erfolgt. Die Ursache der Abweichung von einem idealen Behandlungsverlauf kann dem Benutzer in einer Fehlermeldung angezeigt werden, entweder als selbstständige Fehlermeldung oder zusammen mit der Fehlermeldung, die eine Abweichung der Dilalysance oder Clearance anzeigt. Alternativ oder zusätzlich kann die Ursache der Abweichung in einem Patientendatensatz abgelegt werden, etwa in einem Patientendatensatz in einer Patientendatenbank.

Ein Verfahren zur Zuordnung eines Zeitpunkts in einem Behandlungsabschnitt zu einer Ursache ist im Folgenden in Verbindung mit Figur 5 beschrieben.

Figur 5 zeigt ein Verfahren S5 zum Erkennen einer Ursache einer Abweichung von einem idealen Betriebszustand einer Blutbehandlung in Abhängigkeit von einem Zeitpunkt zu dem die Clearance oder Dialysance signifikant von einem einen idealen Behandlungsverlauf oder einen idealen Betriebszustand des Dialysators repräsentierenden Referenzwert abweicht.

Das Verfahren S5 kann beispielsweise von der Mess- und Kontrollvorrichtung 25 der Figur 1 durchgeführt werden.

In einem ersten Schritt S51 wird dabei ein Zeitpunkt to bestimmt, zu dem der zuvor bestimmte Wert der Dialysance oder Clearance von dem Referenzwert abweicht.

Die Bestimmung des Zeitpunkts kann dabei in einer Weise erfolgen, dass eine Vielzahl von Dialysancemessungen zu regelmäßig aufeinander folgenden Zeitpunkten ti durchgeführt wird und der gewonnene Messwert der Dialysance mit einem dem jeweiligen Zeitpunkt ti entsprechenden Referenzwert verglichen wird. Wird eine signifikante Abweichung zu dem Referenzwert festgestellt, so erfolgt anschließend eine Zuordnung des Messzeitpunkts ti zu einem Behandlungsabschnitt.

Als Alternative kann die Dialysance zu jeweils einem vorbestimmten Zeitpunkt innerhalb eines jeden Behandlungsabschnitts gemessen werden, wodurch eine Zuordnung zwischen Messzeitpunkt und Behandlungsabschnitt vorbestimmt ist.

In einem zweiten Schritt S511 erfolgt die Zuordnung des Zeitpunkts to zu einem bestimmten Behandlungsabschnitt, in dem vorliegenden Beispiel einem ersten, zweiten oder dritten Behandlungsabschnitt S52, S54 oder S56.

Typischerweise liegt der erste Behandlungsabschnitt zu Beginn der Behandlung. In einer vorteilhaften Ausführungsform ist der erste Behandlungsabschnitt das Zeitintervall zwischen dem Startzeitpunkt der Behandlung und 30 Minuten nach dem Startzeitpunkt der Behandlung.

Wird in einem Schritt S52 erkannt, dass der Messzeitpunkts ti in dem ersten Behandlungsabschnitt liegt, so wird in einem Schritt S53 eine Fehlpositionierung der Konnektoren des Dialysators oder eine Fehlpositionierung der Nadeln in Bezug auf die Flussrichtung in dem Gefäßzugang als Ursache einer Abweichung von einem idealen Behandlungsverlauf oder einem idealen Betriebszustand erkannt. Diese Ursachen können zu der Gruppe der bedienerbezogenen Ursachen für eine Abweichung von einem idealen Behandlungsverlauf zusammengefasst werden. Wird die Fehlpositionierung der Nadeln oder des Gefäßzugangs dem Bediener durch eine Fehlermeldung zu Beginn der Behandlung angezeigt, so hat der Bediener zu diesem frühen Zeitpunkt der Behandlung noch die Möglichkeit, die Behandlung zu unterbrechen, um die Fehlpositionierung zu korrigieren. Wenn die Klassifizierung einer Dialysance oder Clearance als signifikant in Abhängigkeit von einer prozentual angegebenen Abweichung erfolgen soll, und der Prozentsatz in Abhängigkeit von dem Behandlungsabschnitt angegeben wird, hat sich für den ersten Behandlungsabschnitt eine Abweichung von mehr als 10% über oder unter dem vorbestimmten Referenzwert als zweckmäßig erwiesen.

Beim Vertauschen der Konnektoren des Dialysators wird das Dialysat im Gleich-strom anstelle wie gewünscht im Gegenstrom zur Blutführung betrieben. Der ma-thematische Zusammenhang für die Clearance ist für beide Fälle bekannt (z.B. J. Sargent und F. Gotch, "Principles and Biophysics of Dialysis" in J. Maher (editor), Replacement of Renal Function by Dialysis, Kluwer, 1989, 3rd edition).

So kann etwa für ausgehend von dem Massentransferkoeffizienten KoA, der Flussrate des Dialysats in den Dialysator und der Blutwasserflussrate in den Dialysator $Q_{Di}$ im Falle der Hämofiltration und des Betriebs des Dialysators im Gegenstrom folgende Beziehung für eine theoretische Abschätzung oder einen theoretischen Wert $K_{HDcounter}$ der Clearance angegeben werden:

$$K_{HDcounter}\left(k0A, Q_B, Q_D\right) := Q_B \cdot \frac{\exp\left[k0A \cdot \left(\frac{1}{Q_B} - \frac{1}{Q_D}\right)\right] - 1}{\exp\left[k0A \cdot \left(\frac{1}{Q_B} - \frac{1}{Q_D}\right)\right] - \frac{Q_B}{Q_D}}$$

Für die Clearance $K_{HDcocurr}$ desselben Dialysators bei der Flussrate des Dialysats in den Dialysator $Q_{Di}$ und der Blutwasserflussrate in den Dialysator $Q_{Di}$ gilt im Gleichstrombetrieb:

$$K_{HDcocurr}\left(k0A, Q_B, Q_D\right) := Q_B \cdot \frac{1 - \exp\left[-k0A \cdot \left(\frac{1}{Q_B} + \frac{1}{Q_D}\right)\right]}{1 + \frac{Q_B}{Q_D}}$$

Die erwartete normierte Abweichung $\Delta_{cocurent}$ zwischen der Clearance läßt sich bei bekanntem k0A-Wert des Filters, Blutfluß $Q_B$ und Dialysatfluss $Q_D$ berechnen.

$$\Delta_{cocurrent}\left(k0A, Q_B, Q_D\right) := 1 - \frac{K_{HDcocurr}\left(k0A, Q_B, Q_D\right)}{K_{HDcounter}\left(k0A, Q_B, Q_D\right)}$$

So beträgt z.B. für einen FX80 Dialysator der Anmelderin bei einem Blutfluss von 300 ml/min und einem Dialysatfluss von 500 ml die Abweichung 32%, d.h. die Clearance bei vertauschten Konnektoren ist um 32% kleiner, als für Gegenstrom erwartet. Bei einem $Q_D$ von 800 ml/min und sonst gleichen Bedingungen beträgt die Abweichung 24%. Wenn die Abweichung zwischen gemessener Clearance und für Gegenstrom erwarteter Clearance in dem Bereich liegt, der für Gleichstrom erwartet wird, kann ein Vertauschen der Kupplungen des Dialysators als Ursache der Störung vermutet werden.

Wird in einem Schritt S54 erkannt, dass der Messzeitpunkt ti in dem zweiten Behandlungsabschnitt liegt, so wird in einem Schritt S55 eine Rezirkulation als Ursache einer Abweichung von einem idealen Behandlungsverlauf oder einem idealen Betriebszustand erkannt.

Typischerweise ist der zweite Behandlungsabschnitt ein mittlerer Behandlungsabschnitt, d.h. ein Behandlungsabschnitt in der Mitte der Behandlung, der den Zeitpunkt 60 Minuten nach dem Beginn der Behandlung umfasst, zum Beispiel etwa 30 - 90 Minuten nach dem Beginn der Behandlung, etwa 45 - 75 Minuten nach dem Beginn der Behandlung oder 55 - 65 Minuten nach dem Beginn der Behandlung. Vorteilhafterweise liegt der zweite Behandlungsabschnitt nach einen Zeitpunkt, an dem bereits ein Druckhaltetest durchgeführt wurde. Wenn die Klassifizierung einer Dialysance oder Clearance als signifikant in Abhängigkeit von einer prozentualen Abweichung erfolgen soll, und der Prozentsatz, in Abhängigkeit von dem Behandlungsabschnitt angegeben ist, hat sich für den zweiten Behandlungsabschnitt eine Abweichung von mehr als 15% über oder unter dem vorbestimmten Behandlungsabschnitt als zweckmäßig erwiesen.

Der zweite Behandlungsabschnitt kann so gewählt sein, dass das (relative) Blutvolumen nahe dem Zustand der Normohydrierung ist und die Alterung des Blutschlauchsatzes in einem Bereich ist, bei dem der angezeigte Blutfluss am besten mit dem tatsächlichen Blutfluss übereinstimmt. Ein Clotting der Membran ist typischerweise zu diesem Zeitpunkt der Behandlung noch nicht eingetreten.

Wenn ein Behandlungsabschnitt nach dem Zeitpunkt zu dem der Druckhaltetest durchgeführt wurde, gewählt wird, kann außerdem ein Effekt der Bildung von Sekundärmembranen am ehesten ausgeschlossen werden.

Da andere Ursachen für eine Abweichung von einem idealen Behandlungsverlauf in diesem Behandlungsabschnitt also am ehesten ausgeschlossen werden können, ist dieser Behandlungsabschnitt besonders geeignet, um eine Rezirkulation als Ursache von einem idealen Behandlungsverlauf festzustellen.

Dem Schritt 55 des Erkennens, dass eine Rezirkulation die Ursache der Abweichung von einem idealen Betriebszustand ist, kann ein Schritt 551 eines Auslösens einer Rezirkulationsmessung mit einem Bluttemperaturmonitor vorausgehen. Bei einer Rezirkulationsmessung mit einem Bluttemperaturmonitor wird zunächst eine Veränderung der Temperatur des Blutes im stromab des Dialysators im venösen Zweig des extrakorporalen Blutkreislauf ausgelöst und anschließend eine Messung der Temperatur im arteriellen Zweig des extrakorpo-ralen Kreislauf stromauf des Dialysators vorgenommen. Die Temperaturveränderung im arteriellen Zweig aufgrund einer vorangegangenen Temperaturänderung im venösen Zweig kann als ein Maß für die Bestimmung der Rezirkulation herangezogen werden.

Diese Rezirkulationsmessung mit dem Bluttemperaturmonitor ist somit eine zweite und von der Messung der Clearance unabhängige Messmethode, die herangezogen werden kann, um eine Rezirkulation als Ursache für eine für eine Abweichung von einem idealen Betriebszustand der Blutbehandlung zu bestätigen oder nicht.

Wird in einem Schritt S56 erkannt, dass der Messzeitpunkts ti in dem dritten Behandlungsabschnitt liegt, so wird in einer vorteilhaften Ausführungsform zunächst ein Zwischenschritt S57 ausgeführt, in dem eine Dämpfungsmessung eines Druckpulses senkrecht zur Membran durchgeführt wird. Dazu kann die Auswirkung einer Druckquelle im extrakorporalen Blutkreislauf, etwa einer Blutpumpe auf einen Druckaufnehmer im extrakorporalen Blutkreislauf mit der Auswirkung im Dialysierflüssigkeitskreislauf verglichen werden. Hierzu kann, wie im Zusammenhang mit der Figur 1 genauer beschrieben das von einem Druckaufnehmer im Dialysierflüssigkeitskreislauf aufgenommene Signal in sein Frequenzspektrum zerlegt und mit dem Frequenzspektrum des von dem Druckaufnehmer im extrakorporalen Blutkreislauf aufgenommenen Signals verglichen werden.

Ergibt die Dämpfungsmessung eine gegenüber einem Referenzwert erhöhte Dämpfung des Druckpulses, so wird in einem Schritt S58 ein Clotting der semipermeablen Membran des Dialysators als Ursache der Abweichung von einem idealen Betriebszustand oder einer idealen Behandlungssituation bestätigt.

Wird der Zwischenschritt S57 nicht durchgeführt, so wird in Schritt S58 unmittelbar nach dem Schritt S56 erkannt, das ein Clotting der semipermeablen Membran des Dialysators vorliegt.

Typischerweise erstreckt sich der dritte Behandlungsabschnitt vom Ende des zweiten Behandlungsabschnitts bis zum Ende der Behandlung, also etwa von 65 Minuten nach Beginn der Behandlung zum Ende der Behandlung, von 75 Minuten nach dem Beginn der Behandlung bis zum Ende der Behandlung, von 65 Minuten nach dem Beginn der Behandlung bis zum Ende der Behandlung oder von 60 Minuten nach dem Beginn der Behandlung bis zum Ende der Behandlung.

Die Feststellung, ob eine signifikante Abweichung von einem Referenzwert vorliegt, kann im Falle des dritten Behandlungsabschnitts vorteilhafterweise in einer Weise erfolgen, dass eine relative Abweichung während des Behandlungsverlaufs bestimmt wird. Dies kann in der Weise geschehen, dass eine in dem zweiten Behandlungsabschnitt bestimmte Abweichung von einem Referenzwert "herausgerechnet" wird, um eine vorhandene Rezirkulation zu berücksichtigen, und für die Bestimmung, ob eine signifikante Abweichung vorliegt nur noch eine weitere Abweichung gegenüber bereits zuvor bestimmten Abweichung berücksichtigt wird. Diese weitere Abweichung wird dabei so verstanden, dass sie auf ein Clotting der semipermeablen Membran zurückzuführen ist.

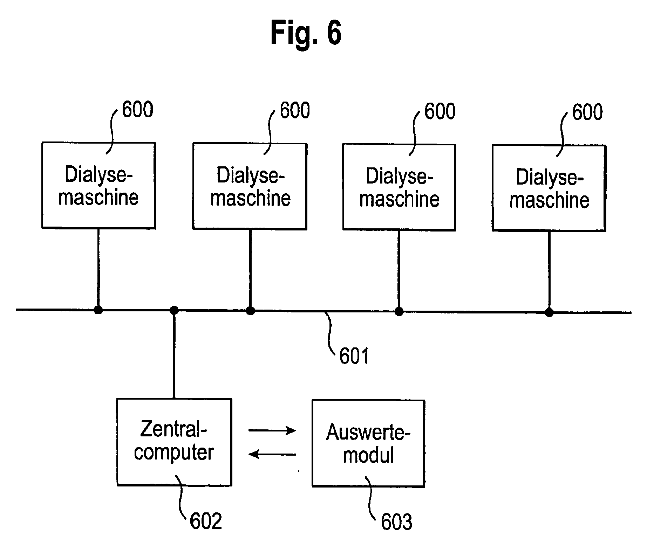

Figur 6 zeigt eine Anzahl von Dialysemaschinen 600 die über ein Netzwerk 601 miteinander und mit einem Zentralrechner 602 verbunden sind. Die Dialysemaschinen 600 entsprechen in Ihrem Aufbau der Dialysiervorrichtung 100 der Figur 1. Der Zentralcomputer 602 ist an ein Auswertemodul 603 angeschlossen, das die Funktionalität der Kontroll- und Auswerteeinheit 25 der Figur 1 oder Teile davon umfasst. Insbesondere ist das Auswertemodul 603 geeignet, die Aktoren und Sensoren der Dialysemaschinen 600 anzusteuern und so das in Figur 4 dargestellte Verfahren und/oder das in der Figur 5 dargestellte Verfahren durchzuführen.

**Patentansprüche**

1.   Vorrichtung (25) zur Erkennung einer Ursache einer Abweichung von einem idealen Betriebszustand oder einem idealen Behandlungsverlauf bei einer extrakorporalen Blutbehandlung, bei der das zu behandelnde Blut in einem extrakorporalen Blutkreislauf (9) eine Blutkammer (3) eines durch eine semi-permeable Membran (2) in die Blutkammer (3) und eine Dialysierflüssigkeitskammer (4) unterteilten Dialysators (1) durchströmt und Dialysierflüssigkeit in einem Dialysierflüssigkeitskreislauf (10) die Dialysierflüssigkeitskammer (4) des Dialysators durchströmt, enthaltend
Mittel zum Verändern einer physikalischen oder chemischen Kenngröße (170; 171) der Dialysierflüssigkeit stromauf der Dialysierflüssigkeitskammer (1) während der Blutbehandlung, Messmittel (174) zum Messen einer physikalischen oder chemischen Kenngröße der Dialysierflüssigkeit stromab der Dialysierflüssgkeitskammer, eine Einrichtung zum Bestimmen eines Wertes der Dialysance oder Clearance aus einer Veränderung der gemessenen physikalischen oder chemischen Kenngröße zu einem bestimmten Zeitpunkt während der Blutbehandlung, **gekennzeichnet durch** eine Einrichtung (25) zum Feststellen einer Abweichung des bestimmten Wertes der Dialysance oder Clearance von einem einen idealen oder komplikationsfreien Behandlungsverlauf repräsentierenden zeitabhängigen Referenzwert der Dialysance oder Clearance, und eine Auswerteeinrichtung (25) zum Erkennen einer Ursache einer Abweichung von einem idealen Betriebszustand in Abhängigkeit von dem bestimmten Zeitpunkt.

2.   Vorrichtung nach Anspruch 1, wobei die Einrichtung (25) zum Bestimmen einer Abweichung des bestimmten Wertes der Dialysance oder Clearance von einem Referenzwert ausgebildet ist, den Referenzwerte aus einer vorangegangenen Blutbehandlung oder auf Basis einer theoretischen Beziehung zu bestimmen.

3.   Vorrichtung nach Anspruch 2, wobei die Einrichtung (25) zum Bestimmen einer Abweichung des bestimmten Wertes der Dialysance oder Clearance von einem Referenzwert ausgebildet ist, den Referenzwert unter Verwendung eines aktuellen Betriebsparameters der Blutbehandlung zu korrigieren oder zu kompensieren.

4.   Vorrichtung nach einem der Ansprüche 1 - 3, wobei die Auswerteeinrichtung (25) angepasst ist, in Abhängigkeit von einem Behandlungszeitpunkt in einem ersten Behandlungsabschnitt eine Fehlpositionierung der Konnektoren des Dialysators oder eine Fehlpositionierung der Nadeln im extrakorporalen Kreislauf als Ursache der Abweichung von einem idealen Betriebszustand zu erkennen.

5.   Vorrichtung nach einem der Ansprüche 1 - 4, wobei die Auswerteeinrichtung (25) angepasst ist, in Abhängigkeit von einem Behandlungszeitpunkt in einem zweiten Behandlungsabschnitt eine Rezirkulation als Ursache der Abweichung von einem idealen Betriebszustand zu erkennen.

6.   Vorrichtung nach Anspruch 5, wobei die Auswerteeinrichtung angepasst ist, eine Veränderung der Temperatur des Blutes im extrakorporalen Blutkreislauf auszulösen, eine Messung der Temperatur im extrakorporalen Kreislauf stromauf des Dilaysators auszulösen, die gemessene Temperatur zur Bestimmung eines Maßes der Rezirkulation zu verwenden und bei der Erkennung der Rezirkulation als Ursache der Abweichung zu berücksichtigen.

7.   Vorrichtung nach Anspruch 5 oder 6, enthaltend Mittel zum Bestimmen eines Zeitpunkts bei dem ein Druckhaltetest durchgeführt wurde, wobei die Auswerteeinheit angepasst ist, in Abhängigkeit von einem Zeitpunkt bei dem zuvor ein Druckhaltetest durchgeführt wurde eine Rezirkulation als Ursache der Abweichung von einem idealen Betriebszustand zu erkennen.

8.   Vorrichtung nach einem der vorangegangenen Ansprüche, wobei die Auswerteeinrichtung angepasst ist, eine Rezirkulation als Ursache der Abweichung von einem idealen Betriebszustand zu erkennen, nachdem zuvor eine Fehlpositionierung der Konnektoren des Dialysators oder eine Fehlpositionierung der Nadeln ausgeschlossen wurde.

9. Vorrichtung nach einem der vorangegangenen Ansprüche, wobei die Auswerteeinrichtung angepasst ist, in Abhängigkeit von einem Zeitpunkt in einem dritten Behandlungsabschnitt ein Clotting der semipermeablen Membran als Ursache der Abweichung von einem idealen Betriebszustand zu erkennen.

10. Vorrichtung nach einem der vorangegangenen Ansprüche, enthaltend Messmittel (34; 33) zur Messung einer Dämpfung eines Druckpulses in dem Dialysator (1), Mittel zum Erzeugen eines Steuersignals zum Auslösen einer Messung der Dämpfung des Druckpulses, wenn zuvor eine Abweichung der Dialysance bestimmt wurde, wobei die Auswerteeinrichtung angepasst ist, eine gemessene Dämpfung des Druckpulses bei der Erkennung eines Clottings der semipermeablen Membran zu berücksichtigen.

11. Blutbehandlungsvorrichtung mit einem durch eine semipermeable Membran (2) in eine Blutkammer (3) und eine Dialysierflüssigkeitskammer (4) unterteilten Dialysator (1), wobei die Blutkammer (4) in einen extrakorporalen Blutkreislauf (9) und die Dialysierflüssigkeitskammer (4) des Dialysators (1) in einen Dialysierflüssigkeitskreislauf (10) geschaltet sind und einer Vorrichtung zur Erkennung einer Ursache einer Abweichung von einem idealen Betriebszustand oder einem idealen Behandlungsverlauf einer extrakorporalen Blutbehandlung nach einem der Ansprüche 1 bis 10.

12. Computerprogrammprodukt enthaltend Teile von Programmcode angepasst zur Ausführung eines Verfahrens zur Erkennung einer Ursache einer Abweichung von einem idealen Betriebszustand oder einem idealen Behandlungsverlauf bei einer extrakorporalen Blutbehandlung bei der das zu behandelnde Blut in einem extrakorporalen Blutkreislauf (9) eine Blutkammer eines durch eine semipermeable Membran (2) in die Blutkammer (3) und eine Dialysierflüssigkeitskammer (4) unterteilten Dialysators (1) durchströmt und Dialysierflüssigkeit in einem Dialysierflüssigkeitskreislauf (10) die Dialysierflüssigkeitskammer (4) des Dialysators (1) durchströmt, enthaltend die folgenden Schritte:

- Verändern einer physikalischen oder chemischen Größe der Dialysierflüssigkeit stromauf der Dialysierflüssigkeitskammer während der Blutbehandlung (S1) und
- Messen einer physikalischen oder chemischen Kenngröße der Dialysierflüssigkeit stromab der Dialysierflüssigkeitskammer (S2),
- Bestimmen eines Wertes der Dialysance oder Clearance aus einer Veränderung der gemessenen physikalischen oder chemischen Kenngröße zu einem bestimmten Zeitpunkt während der Blutbehandlung (S3), **gekennzeichnet durch**
- Feststellen einer Abweichung des bestimmten Wertes der Dialysance oder Clearance von einem einen idealen oder komplikationsfreien Behandlungsverlauf repräsentierenden zeitabhängigen Referenzwert der Dialysance oder Clearance (S4), und
- Erkennen einer Ursache einer Abweichung von einem idealen Betriebszustand der Blutbehandlung in Abhängigkeit von dem bestimmten Zeitpunkt (S5).

13. Computerprogrammprodukt nach Anspruch 12, wobei der Referenzwert aus einer vorangegangenen Blutbehandlung oder auf Basis einer theoretischen Beziehung bestimmt wird.

14. Computerprogrammprodukt nach Anspruch 13, wobei der Referenzwert einer vorangegangenen Behandlung unter Verwendung eines aktuellen Betriebsparameters der Blutbehandlung korrigiert oder kompensiert wird.

15. Computerprogrammprodukt nach einem der Ansprüche 12 bis 14, wobei der bestimmte Zeitpunkt zu dem die Abweichung des Wertes der Dialysance oder Clearance bestimmt wird in einem ersten Behandlungsabschnitt liegt, und als Ursache der Abweichung von einem idealen Betriebszustand eine Fehlpositionierung der Konnektoren des Dialysators oder eine Fehlpositionierung der Nadeln erkannt wird (S53).

16. Computerprogrammprodukt nach einem der Ansprüche 12 bis 15, wobei der bestimmte Zeitpunkt zu dem die Abweichung des Wertes der Dialysance oder Clearance bestimmt wird in einem zweiten Behandlungsabschnitt liegt, und als Ursache der Abweichung von einem idealen Betriebszustand eine Rezirkulation erkannt wird (S55).

17. Computerprogrammprodukt nach Anspruch 16, wobei eine Veränderung der Temperatur des Blutes im extrakorporalen Blutkreislauf ausgelöst wird, eine Messung der Temperatur im extrakorporalen Kreislauf stromauf des Dialysators ausgelöst wird, die gemessene Temperatur zur Bestimmung eines Maßes der Rezirkulation verwenden wird, und das so bestimmte Maß der Rezirkulation bei der Erkennung der Rezirkulation als Ursache der Abweichung berücksichtigt wird

18. Computerprogrammprodukt nach Anspruch 16 oder 17, wobei der Zeitpunkt zu dem eine Rezirkulation als Ursache der Abweichung von einem idealen Betriebszustand erkannt wird, ein Zeitpunkt ist bei dem zuvor ein Druckhaltetest durchgeführt wurde.

19. Computerprogrammprodukt nach einem der Ansprüche 16 - 18, wobei die Rezirkulation als Ursache der Abweichung von einem idealen Betriebszustand erkannt wird, nachdem zuvor eine Fehlpositionierung der Konnektoren des Dialysators oder eine Fehlpositionierung der Nadeln ausgeschlossen wurde.

20. Computerprogrammprodukt nach einem der Ansprüche 11 bis 19, wobei der bestimmte Zeitpunkt zu dem die Abweichung des Wertes der Dialysance oder Clearance bestimmt wird in einem dritten Behandlungsabschnitt liegt, und als Ursache der Abweichung von einem idealen Betriebszustand ein Clotting der semipermeablen Membran erkannt wird (S58).

21. Computerprogrammprodukt nach Anspruch 20, wobei nachdem eine Abweichung des bestimmten Wertes der Dialysance oder Clearance von einem Referenzwert der Dialysance oder Clearance bestimmt wurde, eine Dämpfung eines Druckpulses in dem Dialysator gemessen wird, und die gemessene Dämpfung des Druckpulses bei der Erkennung eines Clottings der semipermeablen Membran berücksichtigt wird.

## Claims

1. An apparatus (25) for detecting the cause for a deviation from an ideal operating state or an ideal course of treatment in an extracorporeal blood treatment, in which the blood to be treated in an extracorporeal blood circulation (9) flows through a blood chamber (3) of a dialyzer (1), which is subdivided by a semipermeable membrane (2) into the blood chamber (3) and a dialysis fluid chamber (4), and the dialysis fluid flows through the dialysis fluid chamber (4) of the dialyzer in a dialysis fluid circulation (10), said apparatus comprising
means for altering a physical or chemical characteristic variable (170; 171) of the dialysis fluid upstream from the dialysis fluid chamber (1) during the blood treatment, measurement means (174) for measuring a physical or chemical characteristic variable of the dialysis fluid downstream from the dialysis fluid chamber, a device for determining a value of the dialysance or clearance from a change in the measured physical or chemical characteristic variable at a certain point in time during the blood treatment, **characterized by** a device (25) for detecting a deviation in the value determined for the dialysance or clearance of a time-dependent reference value of the dialysance or clearance that is representative of an ideal or uncomplicated course of treatment, and an evaluation device (25) for recognizing the cause of a deviation from an ideal operating state as a function of the point in time thereby determined.

2. The apparatus according to claim 1, wherein the unit (25) for determining a deviation in the certain value of the dialysance or clearance from a reference value is formed, to determine the reference values from a previous blood treatment or on the basis of a theoretical relationship.

3. The apparatus according to claim 2, wherein the device (25) for determining a deviation in the value determined for the dialysance or clearance is formed by a reference value, to correct or compensate the reference value by using a current operating parameter of the blood treatment.

4. The apparatus according to any one of claims 1 to 3, wherein the evaluation unit (25) is adapted to detect a false positioning of the connectors of the dialyzer or a false positioning of the needles in the extracorporeal circulation as the cause for the deviation from an ideal operating state as a function of a treatment point in time in a first treatment segment.

5. The apparatus according to any one of claims 1 to 4, wherein the evaluation unit (25) is adapted for detecting a recirculation as the cause for a deviation from an ideal operating state as a function of a treatment point in time in a second treatment segment.

6. The apparatus according to claim 5, wherein the evaluation device is adapted to trigger a change in the temperature of the blood in the extracorporeal blood circulation, to trigger a measurement of the temperature in the extracorporeal circulation upstream from the dialyzer, to use the measured temperature to determine a measure of the recirculation and to take it into account in recognizing the recirculation as the cause of the deviation.

7. The apparatus according to claim 5 or 6, comprising means for determining a point in time at which a pressure-holding test was performed, wherein the evaluation unit is adapted for recognizing recirculation as the cause of the deviation from an ideal operating state as a function of a point in time at which a pressure-holding test was performed in the past.

8. The apparatus according to any one of the preceding claims, wherein the evaluation device is adapted to recognize recirculation as the cause of the deviation from an ideal operating state after previously having ruled out a false positioning of the connectors of the dialyzer or a false positioning of the needles.

9. The apparatus according to any one of the preceding claims, wherein the evaluation device is adapted to recognize a clotting of the semipermeable membrane as the cause of the deviation from an ideal operating state as a function of a point in time in a third treatment segment.

10. The apparatus according to any one of the preceding claims, comprising measurement means (34; 33) for measuring attenuation of a pressure pulse in the dialyzer (1), means for generating a control signal for triggering a measurement of the attenuation of the pressure pulse when a deviation in the dialysance has been determined previously, wherein the evaluation device is adapted for taking into account a measured attenuation of the pressure pulse in recognizing of clotting of the semipermeable membrane.

11. A blood treatment apparatus having a dialyzer (1), which is subdivided by a semipermeable membrane (2) into a blood chamber (3) and a dialysis fluid chamber (4), wherein the blood chamber (4) is switched to an extracorporeal blood circulation (9) and the dialysis fluid chamber (4) of the dialyzer (1) is switched to a dialysis fluid circulation (10), and having an apparatus for recognizing a cause of a deviation from an ideal operating state or an ideal treatment course of an extracorporeal blood treatment according to any one claims 1 to 10.

12. A computer program product, comprising parts of program code adapted for carrying out a method for recognizing the cause of a deviation from an ideal operating state or an ideal course of treatment in an extracorporeal blood treatment, in which the blood to be treated flows through a blood chamber of a dialyzer (1) that is subdivided by a semipermeable membrane (2) into the blood chamber (3) and a dialysis liquid chamber (4) in an extracorporeal blood circulation (9), and dialysis fluid in a dialysis fluid circulation (10) flows through the dialysis fluid chamber (4) of the dialyzer (1), comprising the following steps:

- altering a physical or chemical variable of the dialysis fluid upstream from the dialysis fluid chamber during the blood treatment (S1), and
- measuring a physical or chemical characteristic variable of the dialysis fluid downstream from the dialysis fluid chamber (S2),
- determining a value of the dialysance or clearance from a change in the measured physical or chemical characteristic variable at a certain point in time during the blood treatment (S3), **characterized by**
- determination of a deviation in a determined value for the dialysance or clearance from a time-dependent reference value for the dialysance or clearance (S4) which represents an ideal or uncomplicated course of treatment, and
- recognizing the cause of a deviation from an ideal operating state of the blood treatment as a function of the point in time thereby determined (S5).

13. The computer program product according to claim 12, wherein the reference value is determined from a previous blood treatment or on the basis of a theoretical equation.

14. The computer program product according to claim 13, wherein the reference value from a past treatment is corrected or compensated by using a current operating parameter of the blood treatment.

15. The computer program product according to any one of claims 12 to 14, wherein the point in time thereby determined, at which the deviation in the value for the dialysance or clearance is determined, lies in a first treatment phase, and a false positioning of the connectors of the dialyzer or false positioning of the needles is detected as the cause of the deviation from an ideal operating state (S53).

16. The computer program product according to any one of claims 12 to 15, wherein the point in time thereby determined, at which the deviation in the value for the dialysance or clearance is determined, lies in a second treatment phase, and recirculation is detected as the cause of the deviation from an ideal operating state (S55).

**17.** The computer program product according to claim 16, wherein a change in the temperature of the blood in the extracorporeal blood circulation is triggered, a measurement of the temperature in the extracorporeal circulation upstream from the dialyzer is triggered, the measured temperature is used to determine a measure of the recirculation and the measure of recirculation thereby determined is taken into account in the recognition of recirculation as the cause of the deviation.

**18.** The computer program product according to claim 16 or 17, wherein the point in time at which recirculation is recognized as the cause of the deviation from the ideal operating state is recognized as a point in time at which a pressure-holding test was performed in the past.

**19.** The computer program product according to any one of claims 16 to 18, wherein recirculation is recognized as the cause of the deviation from an ideal operating state after first having ruled out false positioning of the connectors of the dialyzer or false positioning of the needles.

**20.** The computer program product according to any one of claims 11 to 19, wherein the determined point in time at which the deviation of the value for the dialysance or clearance is determined lies in a third treatment phase, and clotting of the semipermeable membrane is recognized as the cause of the deviation from an ideal operating state (S58).

**21.** The computer program product according to claim 20, wherein attenuation of a pressure pulse in the dialyzer is measured after a deviation in the determined value of the dialysance or clearance from a reference value of the dialysance or clearance was determined, and the measured attenuation of the pressure pulse is taken into account in recognition of clotting of the semipermeable membrane.

**Revendications**

**1.** Dispositif (25) de détection d'une cause d'un écart par rapport à un état de fonctionnement normal ou à une évolution de traitement idéale dans un traitement sanguin extracorporel, dans lequel le sang à traiter traverse dans un circuit sanguin extracorporel (9) une cellule à sang (3) d'un dialyseur (1) divisé par une membrane semi-perméable (2) en la cellule à sang (3) et une cellule de liquide de dialyse (4) et du liquide de dialyse traverse dans un circuit de liquide de dialyse (10) la cellule à liquide de dialyse (4) du dialyseur, contenant
des moyens de modification d'une caractéristique physique ou chimique (170; 171) du liquide de dialyse en amont de la cellule à liquide de dialyse (1) pendant le traitement sanguin, des moyens de mesure (174) pour mesurer une caractéristique physique ou chimique du liquide de dialyse en aval de la cellule à liquide de dialyse, un dispositif de détermination d'une valeur de dialysance ou de clairance à partir d'une modification de la caractéristique physique ou chimique mesurée à un certain moment pendant le traitement sanguin, **caractérisé par** un dispositif (25) de constatation d'un écart de la valeur définie de dialysance ou de clairance par rapport à une valeur de référence à dépendance chronologique représentant une évolution idéale ou sans complications du traitement de la dialysance ou de la clairance et un dispositif d'exploitation (25) pour détecter une cause d'un écart par rapport à un état de fonctionnement normal en fonction du moment défini.

**2.** Dispositif selon la revendication 1, dans lequel le dispositif (25) de détermination d'un écart de la valeur définie de dialysance ou de clairance par rapport à une valeur de référence est conçu pour déterminer la valeur de référence à partir d'un traitement sanguin précédent ou sur la base d'une relation théorique.

**3.** Dispositif selon la revendication 2, dans lequel le dispositif (25) de détermination d'un écart de la valeur définie de dialysance ou de clairance par rapport à une valeur de référence est conçu pour corriger ou compenser la valeur de référence en utilisant un paramètre de fonctionnement actuel du traitement sanguin.

**4.** Dispositif selon une des revendications 1-3, dans lequel le dispositif d'exploitation (25) est apte, en fonction d'un moment du traitement, dans une première partie du traitement, à détecter nous un mauvais positionnement des connecteurs du dialyseur ou un mauvais positionnement des aiguilles dans le circuit extracorporel en tant que cause de l'écart par rapport à un état de fonctionnement idéal.

**5.** Dispositif selon une des revendications 1-4, dans lequel le dispositif d'exploitation (25) est apte, en fonction d'un moment du traitement, dans une seconde partie de traitement, à détecter une recirculation en tant que cause de l'écart par rapport à un état de fonctionnement idéal.

**6.** Dispositif selon la revendication 5, dans lequel le dispositif d'exploitation est apte à déclencher une modification de la température du sang dans le circuit sanguin extracorporel, une mesure de la température dans le circuit extracorporel en amont du dialyseur, à utiliser la température mesurée pour déterminer un indice de recirculation et, lors de la détection de la recirculation, à le prendre en compte comme cause de l'écart.

**7.** Dispositif selon la revendication 5 ou 6, contenant des moyens de détermination d'un moment auquel un test de retenue de pression a été effectué, l'unité d'exploitation étant apte, en fonction d'un moment auquel un test de retenue de pression a été auparavant effectué, à détecter une recirculation en tant que cause de l'écart par rapport à un état de fonctionnement idéal.

**8.** Dispositif selon une des revendications précédentes, dans lequel l'unité d'exploitation est apte à détecter une recirculation en tant que cause de l'écart par rapport à un état de fonctionnement idéal après qu'un mauvais positionnement des connecteurs du dialyseur ou qu'un mauvais positionnement des aiguilles a été précédemment exclu.

**9.** Dispositif selon une des revendications précédentes, dans lequel l'unité d'exploitation est apte, en fonction d'un moment d'une troisième partie du traitement, à détecter une coagulation de la membrane semi-perméable en tant que cause de l'écart par rapport à un état de fonctionnement idéal.

**10.** Dispositif selon une des revendications précédentes, contenant des moyens de mesure (34; 33) pour la mesure d'une atténuation d'une impulsion de pression dans le dialyseur (1), des moyens de génération d'un signal de commande pour déclencher une mesure de l'atténuation de l'impulsion de pression si auparavant un écart de dialysance a été déterminé, l'unité d'exploitation étant apte à prendre en compte une atténuation mesurée de l'impulsion de pression lors de la détection d'une coagulation de la membrane semi-perméable.

**11.** Dispositif de traitement sanguin comportant un dialyseur (1) divisé en une cellule à sang (3) et une cellule à liquide de dialyse (4) par une membrane semi-perméable (2), la cellule à sang (4) étant intégrée dans un circuit sanguin extracorporel (9) et la cellule à liquide de dialyse (4) du dialyseur (1) dans un circuit de liquide de dialyse (10) et un dispositif de détection d'une cause d'un écart par rapport à un état de fonctionnement idéal ou à une évolution de traitement idéale d'un traitement sanguin extracorporel selon une des revendications 1 à 10.

**12.** Produit de programmation informatique contenant des éléments de code de programme aptes à réaliser un procédé de détection d'un écart par rapport à un état de fonctionnement idéal ou à une évolution de traitement idéale lors d'un traitement sanguin extracorporel dans lequel le sang à traiter traverse dans un circuit sanguin extracorporel (9) une cellule à sang d'un dialyseur (1) divisée par une membrane semi-perméable (2) en la cellule à sang (3) et une cellule à liquide de dialyse (4) et du liquide de dialyse traverse dans un circuit de liquide de dialyse (10) la cellule à liquide de dialyse (4) du dialyseur (1), comprenant les étapes suivantes :

- modification d'un paramètre physique ou chimique du liquide de dialyse en amont de la cellule à liquide de dialyse pendant le traitement sanguin (Sl) et
- mesure d'une caractéristique physique ou chimique du liquide de dialyse en aval de la cellule à liquide de dialyse (S2),
- détermination d'une valeur de dialysance ou de clairance à partir d'une modification de la caractéristique physique ou chimique mesurée à un certain moment pendant le traitement sanguin (S3), **caractérisé par**
- la constatation d'un écart de la valeur définie de dialysance ou de clairance par rapport à une valeur de référence de dialysance ou de clairance (S4) à dépendance chronologique représentant une évolution idéale ou sans complications du traitement et
- détection d'une cause d'un écart par rapport à un état de fonctionnement idéal du traitement sanguin en fonction du moment défini (S5).

**13.** Produit de programmation informatique selon la revendication 12, dans lequel nous la valeur de référence est définie à partir d'un traitement sanguin précédent ou sur la base d'une relation théorique.

**14.** Produit de programmation informatique selon la revendication 13, dans lequel la valeur de référence d'un traitement précédent est corrigée ou compensée en utilisant un paramètre de fonctionnement actuel du traitement sanguin.

**15.** Produit de programmation informatique selon une des revendications 12 à 14, dans lequel le certain moment où l'écart de la valeur de dialysance ou de clairance est défini se situe dans une première partie du traitement et un mauvais positionnement des connecteurs du dialyseur ou un mauvais positionnement des aiguilles est détecté en

tant que cause de l'écart par rapport à un état de fonctionnement idéal (S53).

**16.** Produit de programmation informatique selon une des revendications 12 à 15, dans lequel le certain moment où l'écart de la valeur de dialysance ou de clairance est défini se situe dans une seconde partie du traitement et une recirculation est détectée en tant que cause de l'écart par rapport à un état de fonctionnement idéal (S55).

**17.** Produit de programmation informatique selon la revendication 16, dans lequel une modification de la température du sang dans le circuit sanguin extracorporel est déclenchée, une mesure de la température dans le circuit extracorporel en amont du dialyseur est déclenchée, la température mesurée est utilisée pour déterminer un indice de recirculation et l'indice ainsi défini de recirculation est pris en compte lors de la détection de la recirculation en tant que cause de l'écart.

**18.** Produit de programmation informatique selon la revendication 16 ou 17, dans lequel le moment où une recirculation est détectée en tant que cause de l'écart par rapport à un état de fonctionnement idéal est un moment où un test de retenue de pression a été précédemment effectué.

**19.** Produit de programmation informatique selon une des revendications 16 - 18, dans lequel la recirculation est détectée en tant que cause de l'écart par rapport à un état de fonctionnement idéal après qu'un mauvais positionnement des connecteurs du dialyseur ou qu'un mauvais positionnement des aiguilles a été précédemment exclu.

**20.** Produit de programmation informatique selon une des revendications 11 à 19, dans lequel le certain moment où l'écart de la valeur de dialysance ou de clairance est défini se situe dans une troisième partie du traitement et une coagulation de la membrane semi-perméable est détectée en tant que cause de l'écart par rapport à un état de fonctionnement idéal (S58).

**21.** Produit de programmation informatique selon la revendication 20, dans lequel, après que l'écart de la certaine valeur de dialysance ou de clairance par rapport à une valeur de référence de dialysance ou de clairance a été défini, une atténuation d'une impulsion de pression dans le dialyseur est mesurée et l'atténuation mesurée de l'impulsion de pression est prise en compte lors de la détection d'une coagulation de la membrane semi-perméable.

Fig. 1

**Fig. 2a**

cDi

QD

cBo

1

4

3

2

cDo

QB   cBi

**Fig. 2b**

c

$\Delta$ Mi

$cDi(1) = cDi(0) + dcDi$

cDi(0)

$cDo(1) = cDo(0) + dcDo$

$\Delta$ Mo

cDo(0)

t, Q*t

EP 2 714 128 B1

# Fig. 3

# Fig. 4

Erkennung einer Ursache einer Abweichung von einem idealen Betriebszustand bei einer extrakorporalen Blutbehandlung ～ M1

Verändern einer physikalischen oder chemischen Kenngröße der Dialysierflüssigkeit stromauf der Dialysierflüssigkeitskammer ～ S1

Messen einer physikalischen oder chemischen Kenngröße der Dialysierflüssigkeit stromab der Dialysierflüssigkeitskammer ～ S2

Bestimmen eines Wertes der Dialysance oder Clearance aus einer Veränderung der gemessenen physikalischen oder chemischen Kenngröße zu einem bestimmten Zeitpunkt ～ S3

Feststellen einer Abweichung des bestimmten Wertes der Dialysance oder Clearance von einem Referenzwert der Dialysance oder Clearance ～ S4

Erkennen einer Ursache einer Abweichung von einem idealen Betriebszustand der Blutbehandlung in Abhängigkeit von dem bestimmten Zeitpunkt ～ S5

# Fig. 5

S5 — ┌─────────────────────────────────────────────────────────┐
│ Erkennen einer Ursache einer Abweichung von einem idealen │
│ Betriebszustand der Blutbehandlung in Abhängigkeit von dem │
│ bestimmten Zeitpunkt │
└─────────────────────────────────────────────────────────┘

S51 — ┌─────────────────────────────────────────────────────────┐
│ Bestimmen eines Zeitpunkts, zu dem ein Wert der │
│ Dialysance oder Clearance von einem Referenzwert abweicht │
└─────────────────────────────────────────────────────────┘

S511 — ┌─────────────────────────────────────────────────────────┐
│ Zuordnen des Zeitpunkts zu einem Behandlungsabschnitt │
└─────────────────────────────────────────────────────────┘

S52 ┌─────────────────────────────────────────────────────────┐
│ der bestimmte Zeitpunkt liegt in einem ersten Behandlungsabschnitt │
└─────────────────────────────────────────────────────────┘

S53 — ┌─────────────────────────────────────────────────────────┐
│ Erkennen einer Fehlpositionierung der Konnektoren des │
│ Dialysators oder eine Fehlpositionierung der Nadeln als Ursache │
│ für die Abweichung von einem idealen Betriebszustand erkannt │
└─────────────────────────────────────────────────────────┘

S54 ┌─────────────────────────────────────────────────────────┐
│ der bestimmte Zeitpunkt liegt in einem zweiten Behandlungsabschnitt │
└─────────────────────────────────────────────────────────┘

S551 — ┌ ─ ─ ─ ─ ─ ─ ─ ─ ─ ─ ─ ─ ─ ─ ─ ─ ─ ─ ─ ─ ─ ┐
Auslösen einer Rezirkulationsmessung mit
einem Bluttemperaturmonitor
└ ─ ─ ─ ─ ─ ─ ─ ─ ─ ─ ─ ─ ─ ─ ─ ─ ─ ─ ─ ─ ─ ┘

S55 — ┌─────────────────────────────────────────────────────────┐
│ Erkennen einer Rezirkulation als Ursache der Abweichung │
│ von einem idealen Betriebszustand │
└─────────────────────────────────────────────────────────┘

S56 ┌─────────────────────────────────────────────────────────┐
│ der bestimmte Zeitpunkt liegt in einem dritten Behandlungsabschnitt │
└─────────────────────────────────────────────────────────┘

S57 — ┌ ─ ─ ─ ─ ─ ─ ─ ─ ─ ─ ─ ─ ─ ─ ─ ─ ─ ─ ─ ─ ─ ┐
Auslösen einer Dämpfungsmessung eines Druckpulses
└ ─ ─ ─ ─ ─ ─ ─ ─ ─ ─ ─ ─ ─ ─ ─ ─ ─ ─ ─ ─ ─ ┘

S58 — ┌─────────────────────────────────────────────────────────┐
│ Erkennen eines Clottings der semipermeablen Membran des Dialysators │
│ als Ursache der Abweichung von einem idealen Betriebszustand │
└─────────────────────────────────────────────────────────┘

# Fig. 6

| Dialyse-maschine | Dialyse-maschine | Dialyse-maschine | Dialyse-maschine |
|---|---|---|---|

600  600  600  600

601

| Zentral-computer | → ← | Auswerte-modul |
|---|---|---|

602  603

**EP 2 714 128 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0911043 A
- WO 2006072271 A
- WO 2008135193 A